Europäisches Patentamt

(19) European Patent Office

Office européen des brevets

(11) Publication number: **0 245 481 B1**

# EUROPEAN PATENT SPECIFICATION

(12)

(45) Date of publication of patent specification: **21.07.93**

(21) Application number: **86907173.8**

(22) Date of filing: **07.11.86**

(86) International application number:
**PCT/US86/02447**

(87) International publication number:
**WO 87/03006 (21.05.87 87/11)**

(51) Int. Cl.⁵: **C12P 7/06**, C12N 15/52, C12N 1/18, C12N 1/00, C12N 15/81

(54) **YEAST STRAINS.**

(30) Priority: **08.11.85 US 796551**

(43) Date of publication of application:
**19.11.87 Bulletin 87/47**

(45) Publication of the grant of the patent:
**21.07.93 Bulletin 93/29**

(84) Designated Contracting States:
**AT BE CH DE FR GB IT LI LU NL SE**

(56) References cited:
**EP-A- 0 162 545**

**EUROPEAN JOURNAL OF BIOCHEMISTRY, vol. 105, 1980, pages 419-424, Berlin, DE; R. SERRANO : "Effect of ATPase inhibitors on the proton pump of respiratory-deficient yeast"**

(73) Proprietor: **GENETICS INSTITUTE, INC.
87 Cambridge Park Drive
Cambridge, Massachusetts 02140(US)**

(72) Inventor: **ROGERS, David, T.
128 Commonwealth Road
Wayland, MA 01778(US)**
Inventor: **SZOSTAK, Jack, W.
308 Commonwealth Avenue
Boston, MA 02115(US)**

(74) Representative: **Vossius & Partner
Siebertstrasse 4 P.O. Box 86 07 67
W-8000 München 86 (DE)**

EP 0 245 481 B1

Rank Xerox (UK) Business Services
(3.10/3.6/3.3.1)

INTERNATIONAL JOURNAL OF BIOCHEMIS-TRY, vol. 7, 1976, pages 389-396, Oxford, GB; K.M. OZANICH et al.: "The relationship of adenosine triphosphate inhibition of phosphofructokinase to the regulation of glycolysis"

A. Lehninger, Biochemistry, second edition. Published 1975 by Worth Publishers, Inc., New York, N.Y., USA, pages 435, 438, 630, 631.

Journal of Molecular Biology, Vol. 186, No. 2, Issued November 1985 (New York, NY, USA) SEDIVY et al. "Fructose bisphosphatase of Saccharomyces cerevisiae: cloning disruption and regulation of the FBP1 structural gene", pp. 307-319

The Journal of Biological Chemistry, Vol. 260, No. 10, Issued May, 1985 (Bethesda, Maryland, USA) VASSAROTII et al. "Isolation of the fructose-1,6-bisphatase gene of the yeast Schizosaccharomyces pombe", pp. 6348-6353

The Journal of Biological Chemistry, vol. 261, No. 9, Issued March, 1986 (Bethesda, Maryland, USA) RITTENHOUSE et al "Amino acid sequence of the phosphorylation site of yeast (Saccharomyces cerevisiae) fructose-1, 6-bisphosphatase" pp. 3939-3943.

Chemical Abstracts, Vol. 84, No. 17, Issued April, 1976 (Columbus, Ohio, USA) HOOGER-GEIDE "Studies on the energy metabolism during the respiratory process by baker's yeast" p. 251, abstract no. 118248z.

Molecular and Cellular Biology, Vol. 4, No. 12, Issued December 1984 (Washington, D.C., USA) HAGUENAUER-T SAPIS et al., "A deletion that includes the signal peptidase cleavage site impairs processing, glycosyla-tion, and secretion of cell surface yeast acid phosphatase", pp. 2668-2675

Gene, Vol. 27, No. 1, Issued March, 1984 (Amsterdam, The Nethrlands) GOFF et al. "Expression of calf prochymosin in Sac-charomyces cerevisiae", pp. 35-46

Biological Abstracts,Vol. 82, No. 10, Issued November 1986 (Philadelphia, Pennsylvania, USA) VOLKERT et al. "Site-specific recom-bination promotes plasmid amplification in yeast" abstract no. 92596. Cell, 1986, 46(4), 541-550

Foundation for Biotechnical and Industrial Fermentation Research Vol. 1, Issued June, 1983 (Helsinki, Finland) HINNEN et al. "High expression and secretion of foreign proteins in yeast" pp. 157-163

## Description

Field of the Invention

This invention relates to yeast, especially bakers or brewers yeast having higher rates of carbon dioxide and ethanol production. For example, a higher rate of carbon dioxide evolution provides more rapid leavening for bakers yeast or a higher rate of ethanol production provides reduced brewing time for brewers yeast. This invention also relates to a novel means for regulating gene expression involving the regulated removal of a transcriptional block.

Background of the Invention

Yeasts are one of the oldest cultivated plants. Their use dates back to about 2000 B.C. Among the various recognized yeast genera, Saccharomyces is of the greatest economic and practical importance, as it is used extensively in the baking, brewing and winemaking industries, as well as in the production of biomass. See, for example, Reed, G., "Yeasts" in the Kirk-Othmer Encyclopedia of Chemical Technology, 24:771-806 (John Wiley & Sons, New York, 1984).

The major, although not the only, function of yeast in fermentation is to provide a source of carbon dioxide and ethanol. Sufficient yeast must be added to dough, wort or other fermentable substrate to obtain the desired rate of carbon dioxide and ethanol production. If a more active yeast were available, less yeast could be used, at a corresponding savings in cost.

Improving the fermentative power of yeast is an ongoing research effort. Both the dried yeast and the moist yeast forms may be improved to increase their carbon dioxide producing ability so as to (1) reduce fermentation time and/or (2) enable the use of less yeast, a considerable cost factor in baking as well as brewing. Improvements in yeast fermentative power also makes the preparation of the more stable active dry yeast (ADY) form attractive. Generally, upon preparation of the ADY from a fresh yeast culture, about 40% of the fermentative ability is lost. Methods for eliminating or reducing this problem are continuously being sought.

One approach to improve dried yeast activity involves a modification of either the drying process, or the drying properties of the yeast strain, so as to prevent the loss of activity which occurs during drying. Process improvements have been made, and classical genetic approaches have been applied to this problem, with moderate success. See for example, U.S. Pat. 3,993,783.

Another approach to solving the problem of low activity dry yeast is described in U.S. Pat. 4,420, 563. Yeast having improved leavening activity, particularly in sweet doughs of high sugar content, was produced by the incremental addition of salts to the growing yeast culture during the latter propagative stages.

The present invention is directed to genetic and chemically induced modifications which increase the carbon dioxide and ethanol producing activity of any yeast strain.

Summary of the Invention

Disclosed herein are processes for increasing the rate of production of carbon dioxide, ethanol, and other fermentation products (eg, citric acid) produced by yeast. Generally, the processes involve reducing the level of ATP in the yeast cell, thereby stimulating glycolysis. In one aspect of the invention the ATP level is reduced by substituting in the yeast genotype (eg, via a single copy or multicopy vector or via cointegration into the yeast genome) a regulatable promoter for the natural promoter of a gene encoding a metabolic enzyme, thus permitting the regulatable expression of the enzyme, thereby permitting the metabolic reaction catalyzed by the enzyme to proceed at the same time as the reverse reaction such that ATP is consumed. In another aspect of the invention, the modification also involves inserting into the yeast genotype a gene encoding a metabolic enzyme, but in this aspect, under the expression control of a promoter permitting constitutive expression of the gene, thereby permitting the metabolic reaction catalyzed by the enzyme to proceed at the same time as the reverse reaction such that ATP is consumed. Further modifications of this invention involve modifying the gene encoding the metabolic enzyme, eg, to prevent or eliminate allosteric or other inhibition or inactivation of the enzyme.

In one embodiment, the enzyme is FDPase. The FDPase gene may additionally be mutagenized such that the codon for Ser-12 of the enzyme is replaced with a codon for an amino acid other than serine or such that allosteric inhibition, eg by AMP and/or fructose-2,6-diphosphate, is reduced or eliminated. In another embodiment, the genetic modification involves modifying a gene for an exocellular APase, e.g. by removing that portion of the gene encoding the leader sequence, such that the modified enzyme remains within the yeast cytoplasm and catalyzes the hydrolysis of intracellular ATP.

One advantage provided by this invention is that the genetic modifications may be "turned on" only during, and preferably at the early stage of, the leavening phase and not during the production-level growth of the yeast. This is a significant advantage in baking yeasts. Alternatively, the genetic modifications may be constitutively expressed such that they are turned on during large scale production, i.e. commercial scale growth of the yeast, for the enhanced production of fermentation products such as ethanol.

Regulation of the genetic modifications may be achieved by using a temperature sensitive promoter or a promoter which is induced by the presence of a specific substance such that the enzyme is expressed only at a predetermined temperature or in the presence of the substance. Alternatively, expression control may be provided by inserting into the yeast genome a FLP gene construct described in greater detail herein.

Vectors useful in these processes include single copy, centromere containing plasmids and multicopy plasmids containing the yeast 2u origin of replication, as well as vectors permitting the cointegration thereof into the yeast genome.

This invention further encompasses processes in which the ATP level is reduced by growing or contacting the yeast with a chemical capable of directly or indirectly inducing ATP consumption. Such chemicals include relatively small organic molecules as well as proteins such as yeast killer toxin.

This invention further encompasses genetically modified yeasts produced by the methods disclosed herein.

Finally, this invention also encompasses a method and vector system for the regulated expression of heterologous genes in yeast, bacteria, and higher eucaryotic cells such as plant and mammalian cells.

Brief Description of the Drawings

Figure 1 illustrates the construction of an integrating yeast plasmid containing the FLP gene expressed from the Gal1 promoter.

Figure 2 illustrates a yeast plasmid containing the Gal promoter fused to the beta-gal coding region from E. coli.

Figure 3 illustrates the induction of $\beta$-galactosidase activity from the Gal1 promoter under glucose limited growth.

Figure 4 illustrates the loss of a heterologous gene by regulated site specific recombination.

Figure 5 illustrates the loss of a transcriptional block from the GPDH promoter expressing the yeast FDPase gene.

Figure 6 illustrates a plasmid containing an expression block bounded by SalI/BglII restriction sites.

Figure 7 illustrates the construction of an expression vector containing origins of replication, and selectable markers for both yeast and E. coli.

Figure 8 sets forth the nucleotide sequence of the cloned yeast FDPase gene.

Figure 9A and B set forth the deduced amino acid sequence of the cloned FDPase enzyme and the amino acid sequence of pig FDPase, respectively.

Figure 10 illustrates the construction of a FDPase cassette for expression from a heterologous promoter.

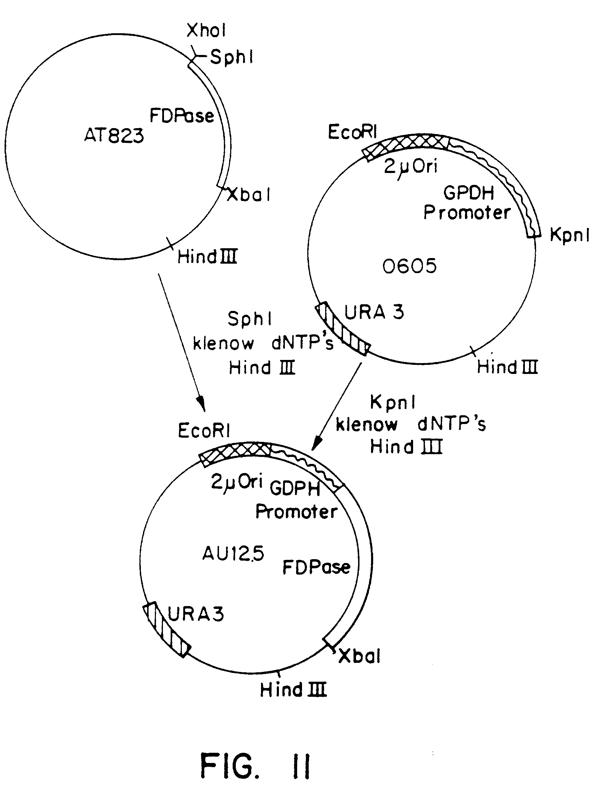

Figure 11 illustrates the synthesis of an expression vector where FDPase is expressed from the GPDH promoter.

Figure 12 illustrates the construction of an amino terminal deletion of FDPase.

Figure 13 illustrates the exchange of the serine residue at the protein kinase recognition site for an alanine.

Figure 14 illustrates carbon dioxide evolution during fermentation of yeast cultures expressing wild type FDPase or amino terminally deleted FDPase.

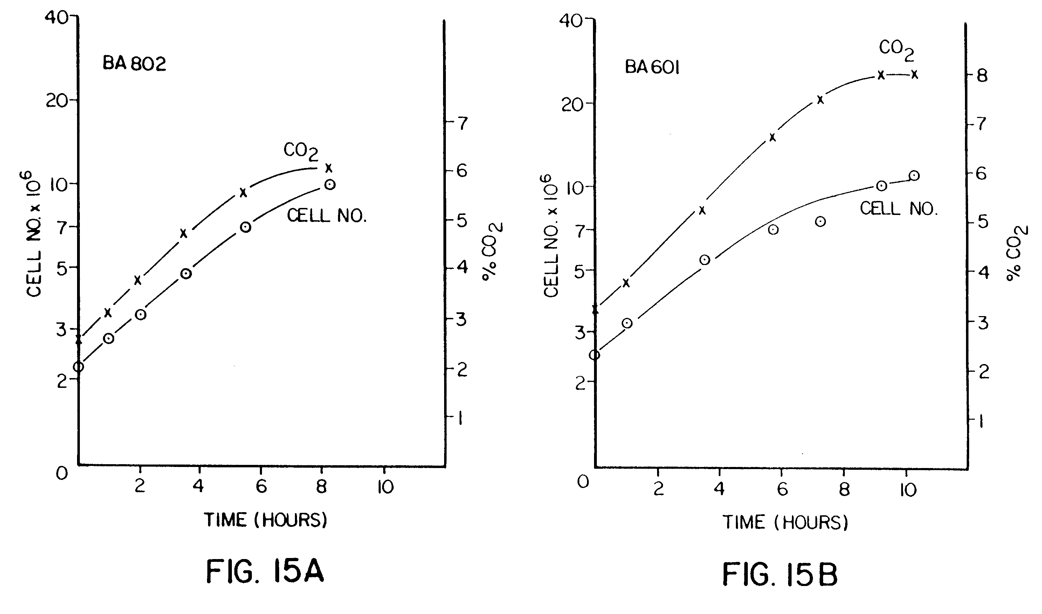

Figure 15 illustrates carbon dioxide evolution during fermentation of yeast cultures expressing FDPase lacking the recognition site for cyclic AMP dependent protein kinase.

Figure 16 illustrates the apparatus used in the gassing test.

Figure 17 illustrates the improvement in gassing power of a strain of yeast expressing the gene for the non-phosphorylated FDPase enzyme. Circles: Yeast strain AT CC 266 75 transformed with plasmid BA601; X: Yeast strain AT CC 266 75 transformed with plasmid BA802

Figure 18 illustrates the introduction of the yeast acid phosphatase promoter into the yeast expression vector.

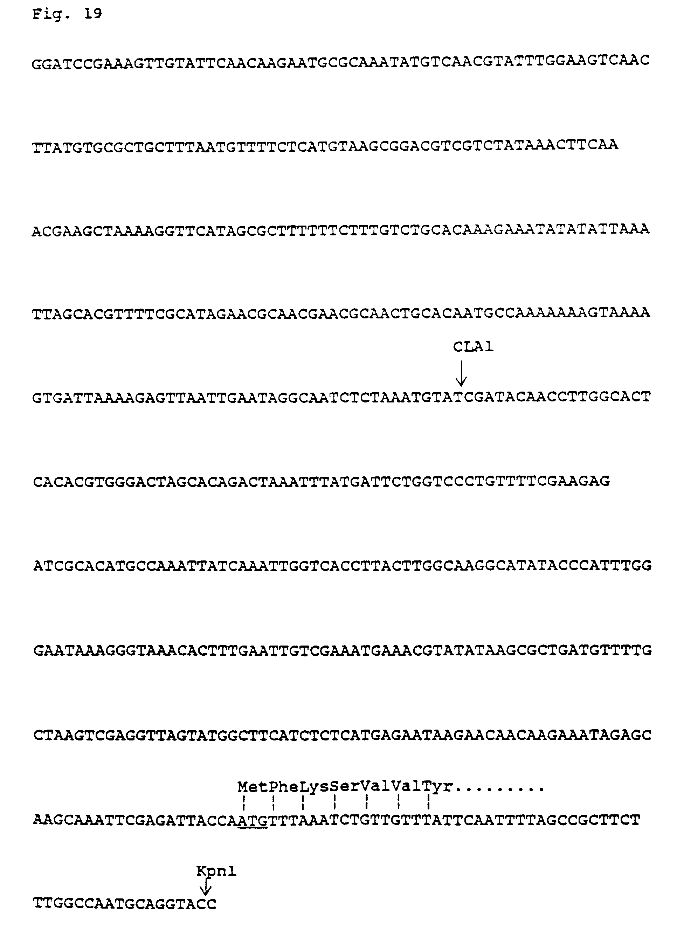

Figure 19 illustrates the DNA sequence for the yeast acid phosphatase promoter and the beginning of the structural gene for acid phosphatase (PHO 5).

Figure 20 illustrates the introduction of a unique Bgl II site at the 3' end of the acid phosphatase promoter.

Figure 21 illustrates the introduction of a restriction site into the acid phosphatase gene for expression of the mature protein.

Figure 22 illustrates the synthesis of an expression vector which expresses the mature acid phosphatase gene from the acid phosphatase promoter.

Figure 23 illustrates the introduction of a yeast centromere into the plasmid containing the modified acid phosphatase gene.

Detailed Description

The glycolytic pathway of yeast has been studied extensively for many years. See for example, Fraenkel, "Carbohydrate Metabolism" in The Molecular Biology of the Yeast Saccharomyces; Metabolism and Gene Expression, Cold Spring Harbor Laboratory, New York. The glycolytic fermentation of sugars is a very inefficient process from the viewpoint of both energy and biomass conversion. Nonetheless, yeasts can grow more rapidly by anaerobic glycolysis, where energy is derived from substrate level phosphorylation, than they can by the oxidative phosphorylation of aerobic growth.

In the course of the research leading to this invention, we have found that the rate of glycolysis is regulated by the yeast cellular levels of adenosine triphosphate (ATP). This discovery is indeed surprising since allthough a regulatory role of ATP in some enzymatic steps has been observed, to our knowledge a regulatory role for ATP in the overall glycolytic pathway has never before been demonstrated or even suggested, despite the extensive study of glycolysis heretofore. The present invention makes use of this discovery by providing modifications for decreasing the cellular levels of cytoplasmic ATP, thus stimulating glycolysis, and thereby increasing the rate of evolution of carbon dioxide and ethanol by the yeast.

As mentioned above, baker's yeast may be improved if its carbon dioxide producing ability during leavening can be increased. Brewers yeast may also be improved by increasing the rate of fermentation (i.e. alcohol production) and reducing the time required for the brewing process. The reduced biomass production during fermentation processes using yeast modified in accord with this invention also improved the fermentation process for ethanol production. This invention provides modifications for making these improvements by regulating the rate of glycolysis through abnormal reduction of the cellular ATP level. By "abnormal reduction of the cellular ATP level" we mean simply a reduction induced by a method of this invention.

Since the enzymes involved in glycolysis are normally in excess, the rate of glycolysis may be limited by the allosteric inhibition of certain glycolytic enzymes by cellular metabolites, including intracellular adenosine triphosphate (ATP) levels, as discovered in the course of the research described hereinafter. Thus, reducing cellular ATP levels in accord with this invention stimulates glycolysis, thereby increasing the rate of carbon dioxide and ethanol output and decreasing biomass production.

In the case of bakers yeast, however, if this alteration of the yeast were to operate during normal growth the yeast would be very inefficient at producing biomass. Thus it would be commercially impractical to grow the yeast due to increased production costs. It is therefore desirable that the genetic modifications of this invention be regulated for baker's yeast production so that it only operates during the leavening (or fermentation) process, not during the production (growth phase) of the yeast itself. One significant advantage of this invention is the ability to turn on the genetic modification and attendant increase in carbon dioxide production such that the increased production accrues at the beginning or early stages of the dough-rising phase. This restraint is not necessary for brewing or ethanol-producing yeasts, however, where production is simultaneously obtained during fermentation.

Several approaches and specific examples thereof for reducing cytoplasmic ATP levels are described below. Generally, these approaches include (i) establishing a regulated futile metabolic cycle which consumes ATP, (ii) introducing or enhancing cytoplasmic ATPase activity in a regulated manner, and (iii) reducing cytoplasmic ATP levels by affecting plasma membrane function. As will become clear from the description which follows, approaches (i) and (ii) above involve introducing an altered gene or altered gene function into the yeast strain via recombinant DNA techniques. Before describing the above-mentioned approaches in further detail, it may be helpful to first describe the tools for effecting such genetic modifications, i.e., suitable vectors, regulated promoters and methods for introducing these changes into baking or brewing strains of yeast.

5

Vectors

The gene responsible for inducing a futile cycle or the cytoplasmic acid phosphatase gene may be cloned under the transcriptional control of a promoter into two types of autonomously replicating expression vector: a single copy, centromere containing plasmid, or a multicopy plasmid. Alternatively the DNA may be introduced into the yeast chromosome by recombination. In addition these vectors contained a selection gene and 3' noncoding regions, as are well known in the art.

The vectors are transformed into a strain of yeast and the yeast cells selected for those containing the vector by a selection protocol as is well known in the art. The selected yeast cells containing the vector, i.e. transformed cells, are grown in a suitable growth media and the promoter induced to start the loss of cytoplasmic ATP.

Suitable selection genes are well known in the art. It is preferred that the selection agent be one that prevents cell growth in the absence of the selection gene. Thus, cells that lose the plasmid in large scale culture do not contain the selection gene and will not over-grow during the fermentation. However, it may be desirable in the commercial production of desired products to avoid the use of certain cell toxins, thereby simplifying the product purification steps. Thus, a desirable selection gene is one that enables transformants to grow in a media lacking a nutrient required for growth of the parental strain. Useful selection genes in the practice of this invention include for example, URA3, LEU2, etc.

The vectors useful herein can be synthesized by techniques well known to those skilled in the art. The components of the vectors such as selection genes, promoters, and the like can be obtained from natural sources or synthesized as discussed below. Basically, components which are available in large quantity (i.e., which are present on natural plasmids, e.g. the 2u plasmid of yeast, or which can be readily synthesized) can be assembled with the appropriate use of restriction enzymes and T4 DNA ligase. If a component is not available in large quantity, it can be amplified by insertion into a bacterial cloning vector such as a plasmid or phage as is well known in the art. Then, with appropriate use of restriction enzymes, large quantities of vector can be obtained by techniques well known in the art by simply culturing the bacteria, digesting its DNA with an appropriate endonuclease, separating the DNA fragments, identifying the DNA containing the component of interest and recovering same. Ordinarily, a transformation vector is assembled in small quantity and then ligated to a suitable autonomously replicating synthesis vector such as a plasmid or phage for production of larger amounts of transformation vector DNA. The well known pBR322 plasmid, for example, can be used in most cases. The synthesis vectors are used to clone the ligated transformation vectors in conventional fashion, e.g. by transformation of a permissive prokaryotic organism, replication of the synthesis vector to high copy number, and recovery of the synthesis vector by cell lysis and separation of the synthesis vector from cell debris. The resulting harvest of synthesis vector can be directly transformed into yeast cells. Many different types of yeast vectors are readily available and may be substituted where appropriate (Parent et al., Yeast 1:83-138 (1985).

Certain vectors including transformation vectors and vectors containing various elements such as specific promoters, the FLP gene and an FDPase gene have been deposited on November 5,1986 in E. coli HB101 with the American Type Culture Collection,12301 Parklawn Drive,Rockville, MD 20852(USA), including the following:

1. AZ402     plasmid contains URA3 transcriptional block flanked by FLP recognition sequences within a Bgl II/Sal I cassette (ATCC No. 67257)

2. AU125     plasmid contains an FDPase gene operatively linked to the GPDH promoter (ATCC No.67256)

3. AT823     plasmid contains an FDPase gene (ATCC No.67258)

4. AR900     plasmid contains the FLP gene operatively linked to the Gal I promoter with restriction sites for the substitution of other promoters for the galI promoter; plasmid provides for regulated expression of the FLP gene (ATCC No.67259)

5. YOp1     plasmid is an illustrative example of a selectable 2u plasmid (ATCC No.67260)

6. BA601     plasmid contains mutagenized (ser-12 to ala) FDPase gene operatively linked to the GPDH promoter (ATCC No.67261)

7. M138     multicopy plasmid for expression of a cytoplasmic (mutagenized) APase gene (ATCC No.67262)

8. N305     similar to M138 except that the plasmid contains a yeast centromere and is therefore a single copy plasmid (ATCC No.67263)

Regulated Promoters

Numerous promoters useful in yeast transformation vectors are known in the art which may be used in the practice of this invention. As discussed in greater detail herein, regulated promoters, many of which are known in the art, are preferred in certain embodiments of this invention. Examples of regulated yeast promoters are those from galactose , maltose, phosphate or nitrogen metabolism, isocytochromes and alcohol dehydrogenase II. A specific example of a regulated promoter is that from the yeast acid phosphatase gene (PHO5). The promoter reportedly acts in response to levels of inorganic phosphate in the media. It is possible that a strong promoter such as that from acid phosphatase (APase) may yield a higher than optimal expression level for certain embodiments of this invention, e.g. futile cycling. The desired regulated promoter can be modulated in several ways. For example, a cloned copy of the acid phosphatase promoter can be mutated in vitro using the method of Shortle (Shortle et al., Proc. Natl. Acad. Sci. USA, 79:1588 (1982)) or small deletion/substitutions within the promoter can be generated by insertion of linkers by known techniques (McKnight and Kingsbury, Science 217:316 (1982), Heffron and McCarthy, Proc. Natl. Acad. Sci. USA 75:6012 (1978)). A pool of DNA fragments containing the mutated acid phosphatase promoter is inserted into a yeast/E. coli shuttle plasmid where the promoter expresses a detectable marker, for example, the beta-galactosidase or beta-lactamase gene from E. coil (Guarente and Ptashne, Proc. Natl. Acad. Sci. USA, 78:2199 (1981); Rose et al., Proc. Natl. Acad. Sci. USA, 78:2460 (1981); Martinez and Casadaban, Mol. Cell. Biol., 3:580 (1983); and Silverman et al., Mol. Cell. Biol., 2:1212 (1982)). Transformed yeast colonies are then screened for the production of the detectable marker, e.g. on media containing 5-bromo-4-chloro-3-indolyl-beta-D-Glactoside (X-gal) where the desired phenotype gives white colonies on high phosphate media and light blue colonies on low phosphate media. Both strong and weak promoters may thus be identified. DNA is obtained from the yeast cells showing a suitable phenotype and transformed into E. coil using standard techniques. Ampicillin resistant colonies must contain the yeast plasmid. Plasmid DNA is made from these E. coil transformants and the acid phosphatase promoter from the plasmids used for expression.

Alternatively, a temperature sensitive regulatory gene may be used. For example, many mutations in the pho R and pho U regulatory genes of the acid phosphatase pathway are found to give constitutive expression at 36°C and normal regulation at 23°C (Ueda et al., J. Bact., 122:911 (1975)). The plasmid of interest containing the PHO5 promoter is transformed into a yeast strain containing a pho R or pho U temperature sensitive mutation and the acid phosphatase promoter is regulated by changing the culture temperature in a high phosphate containing medium. This mode of regulation is also used in conjunction with a weak (mutated) acid phosphatase promoter.

Another method for regulating the modifications of this invention is to use another regulated promoter which is naturally much weaker than the acid phosphatase promoter. Several such promoters have been identified, for example, the promoter from the yeast HO gene. The expression of this promoter is controlled indirectly by mutations in the Sir locus (Rine, "Regulation and Transposition of Cryptic Mating type genes in Saccharomyces cerevisiae", Ph. D. thesis, University of Oregon at Eugene (1979)). In a normal "wild type" cell which has Mat a at the Mating type locus, an alpha cassette HML and the HO allele at the homothallic locus, the HO promoter would be turned on. If the strain carries a Sir mutation however both Mat a and Mat alpha (from HML) are expressed and the HO promoter would be turned off. Therefore a strain carrying a temperature sensitive Sir mutation may be used where the HO promoter is expressed at low temperature but repressed at high temperature.

The regulated promoter is turned "off" during growth of baker's yeast and "on" at the end of fermentation, or in the bread dough, by changing the yeast culture conditions. For example, when using the APase promoter the yeast are grown in the presence of a regulated amount of phosphate so that the culture uses all of the phosphate before the end of fermentation. At this time, the APase promoter is induced by the depletion of phosphate from the fermenter. If a temperature sensitive expression system is used, the culture temperature is set such that the promoter is turned "off" during growth and "on" at the end of fermentation.

Regulated site specific recombination as a mechanism for expression of a futile cycle

One problem for optimizing the expression of an ATP reducing process in baking yeast is the difficulty in identifying a promoter which can be regulated so that it is turned off during growth of the yeast but on during leavening. This is especially difficult since the yeast is used for many different types of baking appilcations where the consistency of the yeast may not be controllable due to different handling conditions which may affect the regulated promoter. If a promoter regulated by the same growth conditions as is normally required for production of $CO_2$, i.e. glycolysis, is used the yeast should be as consistent as the standard baking yeast.

Unfortunately glycolytic genes are not strongly transcriptionally regulated nor are they continually suppressed during growth of the baking yeast. However, triggering the expression of an ATP reducing process by a glycolytic promoter at the end of the growth fermentation would overcome these objections.

A novel approach was therefore devised which allows the yeast to grow up without wasting ATP, but have the ATP reducing process expressed from a glycolytic promoter during the leavening process. This is accomplished using a regulated, site specific recombination event to remove a transcriptional block within the promoter, for example the GPDH promoter, and is described in more detail below. It should be noted that this unusual expression strategy may be used for regulating the expression of a wide variety of genes, including, but not limited to, those encoding enzymes for futile cycling or cytoplasmic phosphatases.

The 2 micron plasmid of yeast has been shown to undergo site specific recombination between two inverted repeats (Hartley and Donelson, (1980) Nature 286:860-864). This recombination is catalysed by a specific recombinase (FLP), whose gene is located on the 2 micron plasmid (Broach and Hicks, (1980) Cell 21:501-508). The GalI promoter is suppressed by glucose and induced by galactose (Yocum et al (1984) Mol. Cell. Biol. 4:1985-1998). Thus if the GalI promoter were used to regulate FLP expression in the baking yeast, providing the natural 2 micron plasmid had been lost, the FLP gene will not be expressed until galactose is present. A growth fermentor is normally run under glucose limitation to prevent the Crabtree effect and the formation of ethanol and to maximize the production of cells. We have found that the GalI promoter is not glucose repressed under these conditions. Addition of galactose to the fermenter thus induces the Gal1 promoter with the consequent expression of the FLP gene. curing the 2 micron plasmid of yeast may be accomplished using, for example, the method of Erhart and Hollenberg, J. Bact. 156:625-635. The FLP gene could also be expressed by another regulateable promoter as described above.

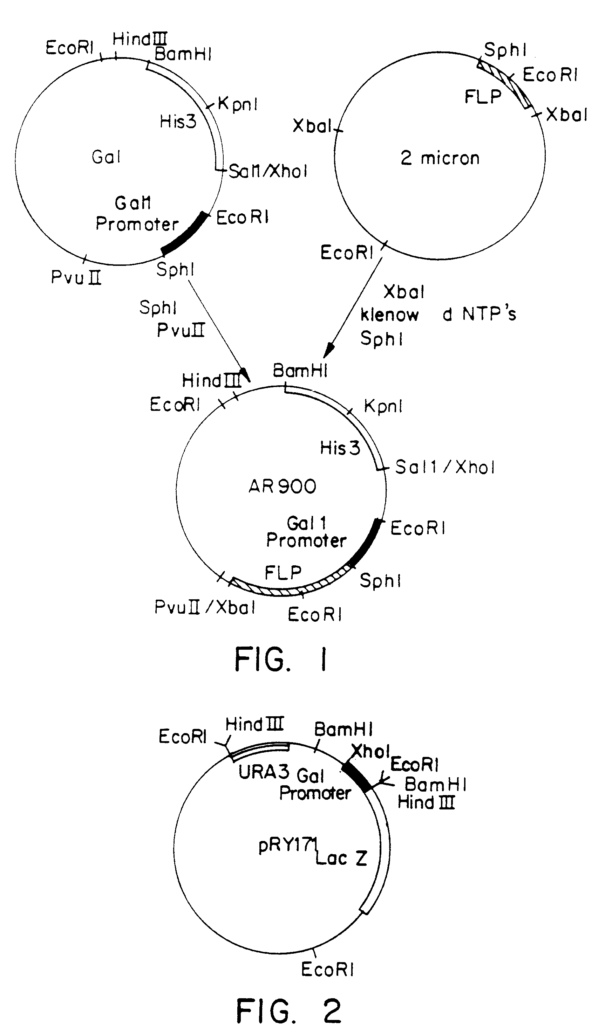

A clone of the FLP gene was therefore isolated and expressed from the regulated GalI promoter. To this end, the 2 micron plasmid of yeast was digested with XbaI and then digested with HindIII and a 1,450 bp XbaI/HindIII fragment isolated by preparative gel electrophoresis. The isolated fragment was then inserted into a conventional yeast plasmid containing the GalI promoter at the PvuII/SphI sites such that the FLP fragment was expressed from the GalI promoter to produce plasmid AR900 (Fig 1). This allowed expression of the FLP gene protein from the Gal1 promoter.

Regulation of the GalI promoter during continuous growth under glucose limitation.

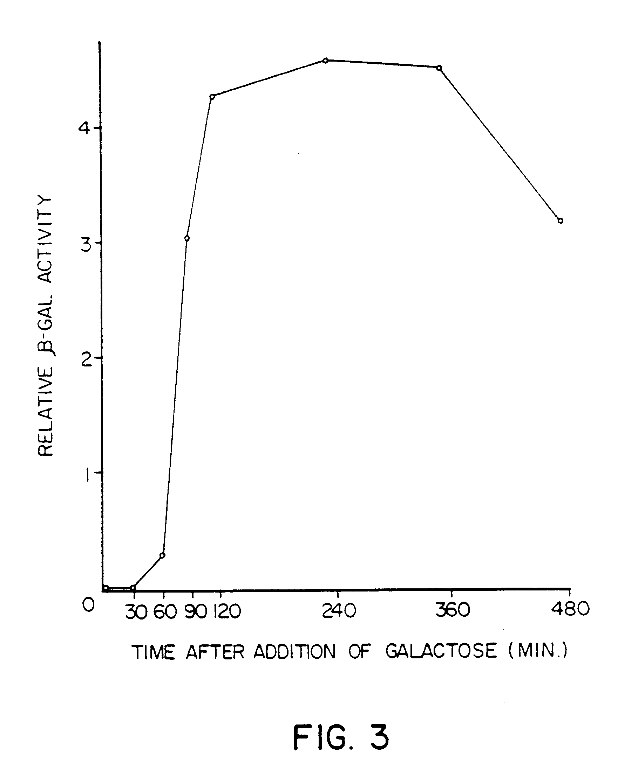

To test further the GalI promoter as a mechanism for regulating the FLP gene, a plasmid (PRY171 Fig 2) containing the Gal promoter fused to the β-galactosidase coding region from E. coli (Parent et al., supra ), was transformed by integration into the genome of a laboratory yeast strain, e.g. KY114. This strain was inoculated into a chemostat where the culture was grown under glucose limitation in yeast minimal media. The culture was stabilized by growth, with a doubling time of 3.5 hours, for 48 hours. Galactose was added to the fermentor at 2% final concentration and samples taken at regular intervals. β-galactosidase activity and protein concentration were measured using standard techniques (Miller, 1972, Experiments in Molecular Genetics. Cold Spring Harbor Laboratory, Cold Spring Harbor, NY; Rose et al., 1981, Proc. Natl. Acad. Sci. 78:2460-2464). As can be seen (Fig. 3) the GalI promoter induced beta-galactosidase activity under conditions or glucose-limited growth when galactose was present.

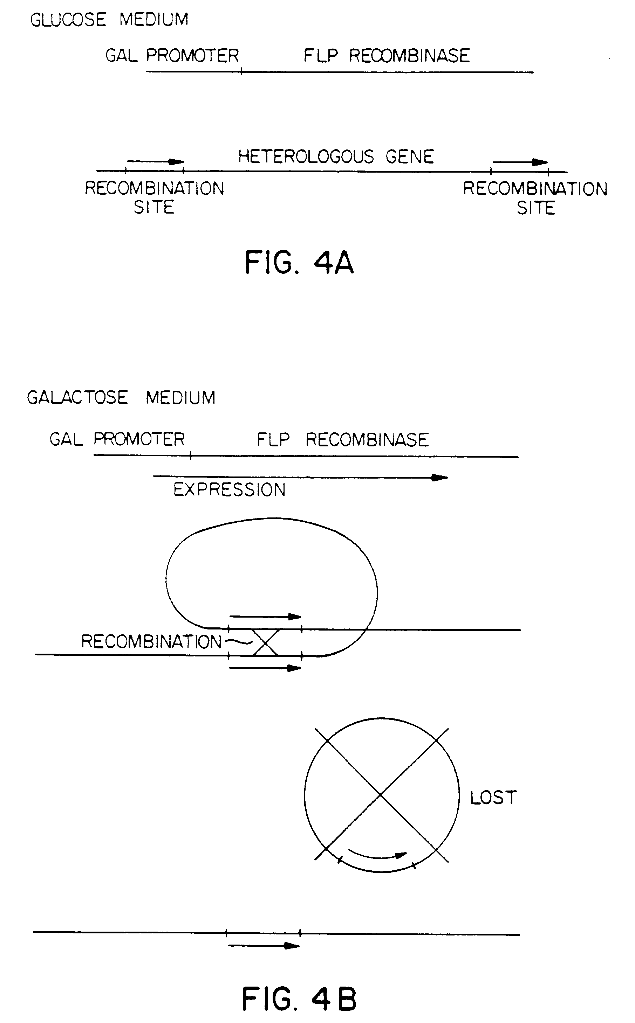

To test the feasibility of using this galactose inducible FLP gene to regulate recombination, a strain of yeast lacking endogenous 2 micron plasmid and which had been previously transformed by integration with a plasmid containing a heterologous gene flanked by tandem repeats from the 2 micron plasmid of yeast (Hartley and Donelson, 1980, Nature 286:860-864; Senecoff et al., 1985, Proc. Natl. Acad. Sci. USA 82:7270-7274; Andrews et al., (1985), Cell 40:795-803) was transformed with a plasmid containing the Gal/FLP fusion gene. If the Gal/FLP fusion works, the strain should express the heterologous gene when grown on glucose (which supresses the GalI promoter). When the strain is grown on galactose, however, the heterologous gene should be excised from the genome by recombination resulting in loss of the gene (Fig. 4). This was indeed found to be the case since on glucose media 97% of the colonies expressed the heterologous gene while on galactose media 0% of the colonies contained the heterologous gene activity. Next an expression block, e.g. a DNA sequence containing a transcriptional block such as the URA3 HindIII fragment, or a silencer region or a transcription terminator (Brand et al., 1985, Cell 21:501-508), is inserted into the promoter, e.g. the GPDH promoter, between the upstream activation sequence and the translational start site. This transcriptional block element is flanked by DNA sequences (inserted as synthetic oligonucleotides, illustrated in Table 1) shown to be recognized by the FLP gene product as substrates for site-specific recombination (Senecoff et al., supra ;Andrews et al., supra). Thus regulation operates by addition of galactose to a growing nonglucose-repressed culture of yeast. Galactose induces the synthesis

# EP 0 245 481 B1

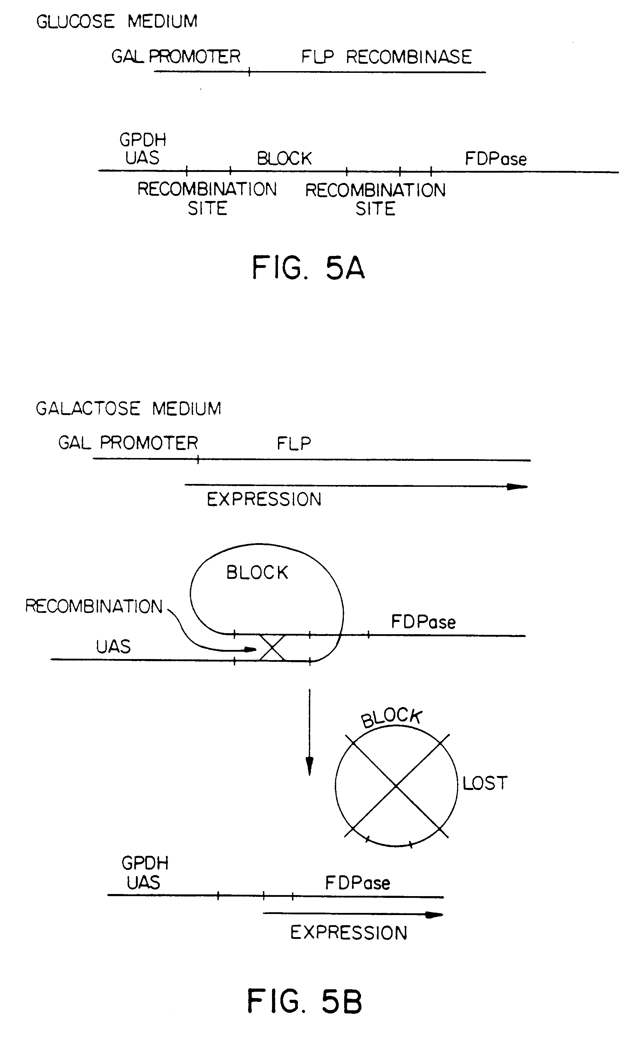

of the FLP protein which catalyzes a recombination event between the DNA sequences flanking the expression block. The recombination event removes the expression block, thereby allowing expression of the heterologous gene, e.g. the gene for FDPase from the GPDH promoter. Fig. 5.

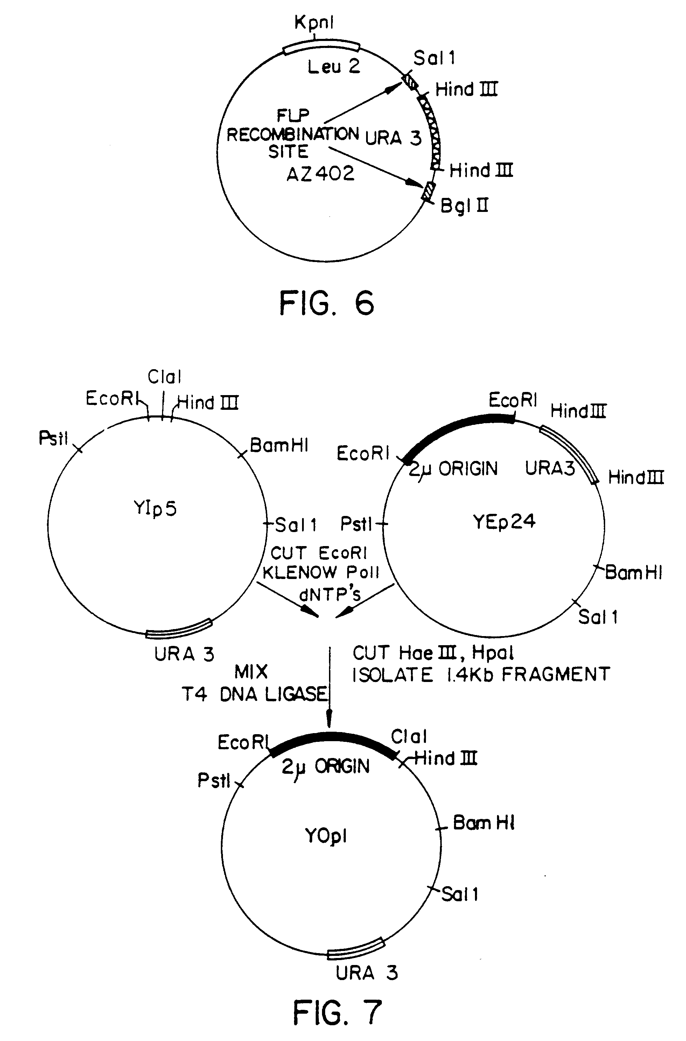

Such a block surrounded by recombination sites illustrated in Fig 6 has been deposited and can be inserted into the promoter of choice by those skilled in the art by first inserting a BglII/SalI linker into the promoter sequence, at a site which allows expression, using site

---

TABLE 1

Secuence for FLP Catalvsed Site Soecific Recombination Site

TCGACGCTTTGAAGTTCCTATTCCGAAGTTCCTA
GCGAAACTTCAAGGATAAGGCTTCAAGGAT    (cont'd on next line)

TTCTCTAGAAAGTATAGGAACTTCAGAGCGCTTA
AAGAGATCTTTCATATCCTTGAAGTCTCGCGAATCTAG

---

directed mutagenesis as described (Zoller and Smith, NAR 10:6487-6500 (1982); Methods Enzymol. 100:468-500 (1983), DNA 3:479-488(1984), and then inserting the transcriptional block from plasmid AZ402 as a BglII/SalI fragment.

This FLP expression system can be used to regulate the expression of virtually any gene in a variety of host cells. Generally, the regulated expression in a host cell of a heterologous DNA sequence may be effected using a two vector system. The first vector contains a DNA sequence encoding a FLP protein (FLP DNA) operatively linked to a regulatable promoter. An example of such a vector is AR900 which contains the FLP gene operatively linked to the Gal I promoter. Similar vectors containing other promoters may be constructed by routine methods by excising the Gal I promoter from AR900 with EcoRI and SphI and substituting therefor any desired promoter, again using conventional techniques and appropriate linkers, if necessary. The second vector contains the heterologous DNA sequence to be expressed, operatively linked to a promoter which contains, inserted therein, an expression block flanked by DNA sequences (flanking DNA) which are recognized by FLP protein. Such vectors may be constructed by conventional means using the SalI - BglII casette from AZ402 together with readily available and/or synthesized components. The SalI - BglII cassette from AZ402 contains the URA3 expression block as a HindIII fragment flanked by FLP-recognized recombination sites. Other expression blocks may be substituted for URA3 using conventional methods, and again, conventional linkers, if necessary. The expression block, e.g. the SalI - BglII cassette from AZ402, is then inserted, with conventional linkers if necessary, into the promoter region of a vector containing the heterologous DNA sequence operatively linked to a promoter such that expression is blocked. Suitable insertion may be readily confirmed empirically by observation of the phenotype of cells transformed with the vector and/or by monitoring the culture medium for the absence of the expression product. Transformation of a host cell with both the first and second vectors described above yields an expression system wherein the FLP protein produced by expression of the FLP DNA in the first vector catalyzes a recombination between the flanking DNA of the second vector, thereby removing the expression block and allowing expression of the heterologous DNA sequence. Naturally, when using any specific host cell, the vectors should contain any genetic elements required by the particular host, as is well known in the art.

Using the above-described vectors and regulated promoters we have produced yeast strains characterized by higher rates of $CO_2$ production, and have produced such strains by introducing into the host strain (i) an ATP-consuming futile cycle and, in another embodiment of the invention, (ii) enhanced cytoplasmic ATPase activity.

9

Genetic Modification of Baking and Brewing Yeast Strains

Baking and brewing yeast strains present a more difficult substrate for transformation than laboratory strains since they are polyploid and do not generally contain auxotrophic markers which can be used for the selection procedures which are well known in the art.

However a modified or a heterologous gene/promoter construct such as the FDPase gene linked to the GPDH promoter, discussed below, can be introduced into the strain of yeast used for baking by using dominant drug resistant genes such as the antifungal agents gentamycin (G418) (Jiminez and Davies, Nature 287:869 (1980))or hygromycin B (Gritz and Davies, Gene 25:179-188 (1983)). As an example of this approach, a resistance gene coding for aminocyclitol phosphotransferase (ACPT) is carried by the bacterial transposon TN601 which confers resistance to G418, but its promoter is weak and therefore is only partially effective at conferring resistance. Jimenez and Davies, supra. The promoter is exchanged for a yeast promoter (e.g., the yeast glyceraldehyde phosphate dehydrogenase promoter). A plasmid is then constructed containing the desired gene/promoter construct with its natural chromosomal flanking regions together with the TN601 ACPT described above. This plasmid does not contain a yeast origin of replication. The strain of bakers yeast is transformed with this plasmid and transformants selected on G418 plates. The plasmid copy of ACPT can only be stably maintained if the plasmid is integrated into the yeast genome at the natural gene locus. This results in a tandem duplication of the gene (e.g. FDPase) separated by the plasmid and ACPT sequences. Growth of these transformants in the absence of G418 allows for the loss of the plasmid by "looping out" leaving behind either the "wild type" or the introduced sequence. These G418 sensitive clones are then screened by Southern hybridization of genomic DNA using oligonucleotide probes for the presence of the heterologous construct using standard techniques.

(i) ATP-consuming Futile Cycles

One genetic modification for reducing cellular ATP levels is the use of a normal metabolic pathway in an abnormal manner to consume ATP, so that glycolysis is stimulated. Most preferably, the chosen pathway is cyclic, so that its abnormal use results in no significant net accumulation or depletion of any required metabolic intermediate, substrate or product. Many metabolic pathways in yeast are capable of running in opposite directions depending on the growth conditions or requirements of the cell. For example, metabolite "A" may be converted into metabolite "B", or "B" into "A", as required by the cell. Causing such a pathway to run in both directions at the same time results in no net accumulation or loss of metabolites but does, however, consume the energy required to run the pathways, and in this respect is a "futile" cycle. Of course, other futile cycles involving additional steps can also be used (e.g. A→B→C→A). Transcriptional, translational and post-translational controls can be used to turn on or off such a futile cycle, where, for every revolution of the cycle, one molecule of ATP is consumed without producing any net accumulation of the product or loss of substrate. In bakers yeast, the metabolic change necessary to reduce ATP levels and thereby stimulate glycolysis is preferably regulated so that it is operable only during leavening.

A preferred futile cycle for consuming ATP is: fructose-6-phosphate→ fructose-1,6-diphosphate→ fructose-6-phosphate. The enzymes involved in this pathway, phosphofructokinase and fructose-1,6-diphosphatase (FDPase or FBPase) have been extensively characterized (Bloxham and Lardy, The Enzymes, Vol. 8, Boyer, Ph.D., Ed., pp 239-278 (1973); Uyeda, Adv. Enzymology, 48, 193-244, (1979); Foy and Bhattachargee, Arch. Microbiol., 129, 215-220 (1981); Funayama et al. (1979)). By cloning the gene for FDPase and exchanging its promoter for one which is regulated, the gene is expressed at will. We have found that expressing this gene during growth on glucose is sufficient to accomplish a considerable loss of ATP and a consequent increase in the rate of glycolysis. Optimizing the FDPase-driven futile cycle requires understanding the natural regulation of FDPase. For the sake of clarity, a brief discussion of FDPase regulation is provided.

FDPase Regulation

The regulation of FDPase has been studied by a number of researchers. In order to prevent futile cycling between the synthesis and hydrolysis of fructose-1,6-diphosphate in wild type yeast this enzyme is rapidly inactivated when glucose is added to cells growing on non-fermentable carbon sources. This inactivation of FDPase appears to occur in three stages. An initial inhibition of enzyme activity is accomplished by allosteric regulation (Lenz and Holzer, F.E.B.S. Lett., 109, 271-274 (1980); Wolf and Holzer, Transport and Utilization of Aminoacids, Peptides and Proteins by Microorganisms, Payne, J.W., Ed., John Wiley, Chinchester, 1980; Tortora et al., Biochem. Biophys. Res. Comm. 100, 688-695 (1981)).

When glucose is added to yeast cells, the concentration of fructose-2,6-diphosphate rises within seconds from undetectable levels to concentrations of several uM, enough to partially inhibit FDPase. The mechanisms that regulate the synthesis of fructose-2,6-diphosphate are unclear (Gancedo et al., J Biol. Chem, 258 5998-5999 (1983)). After the initial rapid inhibition, a second step involving phosphorylation of the FDPase occurs over a period of several minutes (Muller and Holzer, Biochem. Biophys. Res. Comm., 103 926-933 (1981); Tortora et al., supra; Purman et al., Biochem. Biophys. Res. Comm., 107 1482-1489 (1982)). The state of phosphorylation of FDPase is controlled by a specific kinase and a specific phosphatase. The phosphorylation occurs at a particular serine residue (Muller and Holzer supra; Mazon et al., J. Biol. Chem., 257 1128-1130 (1982)). The modified FDPase is less active than the unmodified enzyme. Finally, the phosphorylated enzyme appears to be a substrate for a specific protease which catalyzes an irreversible inactivation of the FDPase, over a period of about an hour.

## Genetic Modifications for Regulating FDPase

There are several genetic approaches that are applicable to block this inactivation of FDPase. Mutants which do not synthesize fructose-2,6-diphosphate, or which have an FDPase that does not bind the inhibitor, block the inactivation cascade at the beginning. Site specific mutagenesis of the serine that otherwise becomes phosphorylated yields an enzyme resistant to the second and third steps. Some enzyme activity remains after the initial partial inhibition by fructose-2,6-diphosphate (Lenz and Holzer, supra; Wolf and Holzer, supra; Tortora et al., supra). This enzyme activity is enough to cause significant futile cycling.

We have identified the site of phosphorylation ($Ser_{12}$) in FDPase since it is the only consensus recognition site for cAMP dependent protein kinases present in the cloned FDPase gene ($Arg_9.Arg_{10}.Asp_{11}.Ser_{12}$) and have readily altered it by conventional site specific mutagenesis. (Zoller and Smith, supra). We have found that an amino acid substitution for the serine to prevent phosphorylation is sufficient to produce enough enzyme activity to cause a significant level of futile cycling. However the enzyme is also inhibited by high concentrations of AMP (Taketa and Pogell, J. Biol. Chem., 240 651-662 (1965)). For further optimization the enzyme (which is already somewhat resistant to AMP inhibition by virtue of the substutition at $Ser_{12}$) may additionally be altered at its AMP binding site to overcome this inhibition. The binding site of AMP on the enzyme has been characterized. To achieve enhanced enzymatic activity for the futile cycle this site may be mutated in vitro and reintroduced into yeast and the loss of inhibition by AMP indirectly assayed. On plates a mutant form of the enzyme no longer inhibited by AMP allows the yeast to grow normally on gluconeogenic carbon source but very poorly on glucose, thus permitting a convenient assay for such a modification.

Since the enzymes involved in this futile cycle are fairly major yeast proteins, this pathway is sufficient to consume a considerable amount of ATP. The stimulation of glycolysis is "fine tuned" to give any desired level of carbon dioxide output by changing the copy number of the altered fructose diphosphatase gene, the strength of the promoter or the promoter used in the FDPase or FDPase-variant expression vector as described.

## Alternative Futile Cycles

There are a surprisingly large number of alternative futile cycles which could be engaged to consume ATP. For example, the conversion of phosphoenolpyruvate to pyruvate by pyruvate kinase can be reversed (Katz, J. and Rognstad, R., Cur. Top. Cell. Reg., 10 237-289 (1976) and Reeves, R.E., Biochem. J. 125 531 (1971)). Alternatively many other futile cycles will waste ATP including those found in amino acid biosynthesis and degradation, polyphosphate synthesis and degradation, fatty acid biosynthesis and pyrimidine biosynthesis. Since all are strictly controlled at the transcriptional level, a futile cycle may be induced by changing the regulation of the enzymes involved by changing their promoters. However, such cycles have additional regulatory mechanisms. Generally, the additional regulation involves feedback inhibition or allosteric modulation of enzyme function by intermediates or energy metabolites. If desired, such allosteric binding sites may be modified to eliminate or reduce allosteric inhibition in analogous fashion to the methods described herein in the illustrative case of FDPase. Other classes of regulation involve the sequestering of one of the enzymes in an organelle, where substrate availability can be controlled, or in the trapping of unstable intermediates in an enzyme complex, allowing them to be quickly converted to a stable intermediate. All of these pathways are potential inducible ATP hydrolysing processes and may therefore be used to consume ATP via appropriate genetic modification.

Where desired the quantity of ATP consumed by the modified cell may be regulated to maximize $CO_2$ and ethanol production. This can be accomplished by using a stronger or weaker promoter or modulating its activity, or by using a temperature sensitive regulatory gene whose degree of regulation is dependent upon the temperature of the culture as described above.

(ii) Introduction of Enhanced Cytoplasmic Acid Phosphatase Activity

An alternative modification for regulating the rate of glycolysis involves producing a cytoplasmic acid phosphatase to hydrolyze organic phosphates including ATP. The normally exocellular acid phosphatase of the yeast Saccharomyces cerevisiae is an inducible non-specific phosphatase located in the periplasm. The gene for this enzyme has been recently cloned and characterized. (See Rogers et al., Proc. Nat'l. Acad. Sci., U.S.A., 79 2157-2161 (1982)). Preventing the phosphatase from being secreted into the periplasm of the cell (e.g., by genetic modification removing the enzyme's secretory leader) will result in dephosphorylating organic phosphates in the cytoplasm, including ATP. However, this non-specific phosphatase will also dephosphorylate other important organic phosphates causing serious damage to the metabolism of the cell. The level of "trapped" cytoplasmic phosphatase must therefore be strictly controlled. For example it was found that the natural promoter for yeast acid phosphatase (PHO5) expresses too much ATPase activity when fully induced to achieve an increase in the rate of glycolysis. To have a regulated method of hydrolysing ATP by a cytoplasmic acid phosphatase, a weaker promoter is preferably used such that it has the desired effect when fully induced or the basal level of an inducible promoter can be used as illustrated below. This can be accomplished in several ways, examples of which have been described above.

(iii) Modification of Plasma Membrane Function

Another embodiment of this invention involves the plasma membrane ATPase, which uses over one third of the ATP generated by fermentation in order to regulate the concentration of ions in the cytoplasm. This is the major pathway for ATP hydrolysis in the cell, stimulation of this pathway may thus be used to reduce the cellular levels of ATP and thereby stimulate fermentation ( i.e. ethanol and carbon dioxide production).

There is some evidence that some drugs which affect the plasma-membrane proton pump induce the cell to stimulate fermentation (Serrano, Eur. J. Biochem., 105:419 (1980)). The stimulation found is very low. The significance of this finding appears to have been overlooked and would be discounted as within experimental error without further work. The reasons for this stimulation are not completely clear since other drugs (i.e. dicyclohexylycarbodiimide and diethylstilbestrol), which also disrupt the plasma membrane proton gradient do not cause a stimulation of fermentation. One explanation, supported by this work, is that dicyclohexylcarbodiimide and diethylstilbestrol inhibit the plasma-membrane ATPase and therefore do not reduce cellular ATP levels. Considerable damage to the general metabolism of the cell may also be inflicted by disruption of other ATP dependent membrane functions. Dinitrophenol appears to act by directly disrupting the proton gradient across the plasma membrane. This stimulates the plasma membrane ATPase and may thereby reduce the levels of cellular ATP.

An alternative method of stimulating the plasma membrane ATPase would be by the use of small proteins such as ribonuclease (Alper et al., (1967) J. Bact. 93:759-765; Yphantis et al., (1967) J. Bact. 94:1509-1515) or yeast killer toxin (Bussey and Sherman, (1973), Biochimica et Biophysica Acta, 298:868-875).

The invention will be further illustrated with reference to the following Examples, which are purely exemplary, and should not be taken as limiting the true scope of the present invention, which is set forth in the claims.

EXPERIMENTAL EXAMPLES

Materials

All DNA restriction and metabolism enzymes were purchased from New England Biolabs. These enzymes were used in conditions and buffers described by New England Biolabs, except mung bean exonuclease which was obtained from PL Biochemicals and used as described. ATP and the deoxynucleoside triphosphate (dNTP's), i.e. dATP, dGTP, dCTP and dTTP, were purchased from PL Biochemicals and [$^{32}$P] was obtained from New England Nuclear Corporation.

Ligation reactions were carried out as described by Maniatis et al., Molecular Cloning, A Laboratory Manual, Cold Spring Harbor Laboratory, Cold spring Harbor, N.Y. (1982), the disclosure of which is incorporated herein by reference, using the buffer described at page 246 thereof and using a DNA concentration of 1-100 ug/ml, at a temperature of 23°C for blunt ended DNA and 16°C for "sticky ended" DNA. Electrophoresis was done in 0.5-1.5% agarose gels containing 90 mM Tris-borate, 10 mM EDTA.

After DNA digestion restriction enzymes were inactivated by heating to 65°C for 10 minutes. When performing sequential reactions the DNA was precipitated with 70% ethanol after each step. After "filling in" a restriction site by reaction with the large fragment of DNA polymerase (Klenow) and the four dNTP's, the reaction mixture was made 10mM magnesium chloride and an equal volume of 5M ammonium acetate was added. The DNA was precipitated with 2 volumes of ethanol at -20° and the DNA pelleted by centrifugation at 4°C for 10 minutes in an Eppendorf microfuge. The ethanol was poured off and the pellet dissolved in 10ul/ug DNA of 0.2M sodium acetate. The DNA was re-precipitated with 2 volumes of ethanol and centrifuged as before. The DNA pellet was dried under vacuum before proceeding to the next step in the construction. Synthetic oligonucleotides were kinased as described in Maniatis et al., supra and annealed by heating to 65°C and slow cooling to 4°C before use.

DNA preparation and transformation

Purification of "super coiled" plasmid DNA from E. coli and the transformation of E. coli was as described in Maniatis et al, 1982, supra. Transformation of yeast was as described by Hinnen et al, Proc. Natl. Acad. Sci. USA, 75, pp 1929-33 (1978), except that 1.2M Sorbitol was used instead of 1.0M. Small scale plasmid preparation for screening transformed bacteria was essentially that described (Maniatis et al., 1982, supra; Holmes and Quigley, Anal. Biochem. 14, p.193 (1981)) except that the RNAse digestion was performed after the restriction enzyme digestion by adding 1 ul of a 1 mg/ml solution of RNAse (Boehringer Mannheim) to the well of the agarose gel just before electrophoresis.

Strains and Media

E. coli strain HB101 was used for all bacterial transformations. Yeast strains DB745 (Botstein et al., Gene 8, pp. 17-24 (1979)), KY114 or ATCC 26675 were used. E. coli were grown in LB media with or without ampicillin (49 ug/ml) as described (Maniatis et al., 1982, supra). Prior to transformation, yeast were grown at 30°C in media containing 1% yeast extract (Difco), 1% Bacto Peptone (Difco) and 2% glucose. Yeast minimal media contained 5 gm ammonium sulfate, 10 gm glucose, 40 mg adenine, 60 mg leucine, 2ug biotin, 400 ug calcium pentothenate, 2 ug folic acid, 2 ug inositol, 400 ug niacin, 100 ug p-aminobenzoic acid, 400 ug pyridoxine hydrochloride, 500 ug boric acid, 40 ug copper sulphate, 100 ug potassium iodide, 200 ug sodium molybdate, 400 ug zinc sulfate, 500 mg magnesium sulphate, 100 mg sodium chloride, 100 mg calcium chloride and 1 gm (high phosphate media) or 10mg (low phosphate media) potassium phosphate (monobasic) per liter. For induction of the acid phosphatase promoter, cells were pregrown at 30°C on high phosphate yeast minimal media, washed free of phosphate, and transferred to low phosphate yeast minimal media to resume growth at 30°C. Maximum induction occurred 8 to 12 hours after transfer.

For the dinitrophenol experiments the yeast strain used in this study was Fleishman's active dry yeast. The yeast was grown in CO containing 1% yeast extract (Difco), 1% Bacto peptone (Difco), 0.05M di-Potassium phosphate and titrated to pH 5.6 with citric acid. The media was made up in 10 liter batches in 15 liter carboys and autoclaved for 2 hours at 121°C. After cooling, sterile glucose was added to 2% (w/v). Antifoam was added every 24 hours to prevent foaming. Dinitrophenol was added directly to the 15 liter carboy of media just before connection of the media feed to the chemostat and to the culture vessel to make the same concentration in the media feed. Dinitrophenol was made up in stock solution in ethanol. Cells were grown up overnight in CO before innoculation into the chemostat. Chemostat culture were grown in a New Brunswick Scientific model F-2000. The culture vessel had been adapted by the addition of a side arm to give a working volume of one liter. Spent culture was allowed to flow out of the vessel by means of a submerged open tube to prevent loss of the antifoam which tended to stay on the surface of the culture.

The fermentor was run at 30°C and an agitation setting of 4. Nitrogen was continuously bubbled through the vessel at a rate of 500 cc /min and the off gas passed through a moisture trap of Dry-Rite and into a Perkin Elmer Mass Spectrometer gas analyzer for the measurement of carbon dioxide.

In continuous culture, media was fed at a rate of 250 ml/hr using a Pharmacia model M3 pump. When carbon dioxide measurements were being taken, all settings, volumes and temperature were checked and adjusted if necessary. Media feed, temperature and agitation were found to be fairly stable, however, the nitrogen gas feed varied by as much as 10% over a four hour period. Therefore, the output from the gas analyzer was fed into a chart recorder and the rate of flow of nitrogen adjusted manually over a two hour period. The rate of flow of nitrogen and the level of carbon dioxide in the off gas was checked for stability over this period before taking measurements of cell density in the culture. The culture was therefore demonstrated to be stable within the limits of detection before measurements were taken. This was born out by the reproducibility of the data. Culture density was measured by dilution of the culture ten fold in water and reading the density in a Bausch and Lomb Model Spectronic 20 at 600 nm.

## Vector Construction

To minimize the size of the expression plasmid and to reduce the number of restriction sites, a plasmid was constructed which contained the uracil 3 gene (URA3) as a selection gene and the 2u origin of replication. Alternatively, another yeast plasmid could be used such as YEp24 or YEp13 or equivalent (Parent et al., supra). Our plasmid was derived from YIp5 (Botstein et al., supra) with the addition of a HaeIII/HpaI fragment, containing the origin of replication from the 2u plasmid of yeast. The plasmid, YOpI (Fig. 7), was constructed by introducing the 2u origin into the EcoRI site of YIp5. Plasmid DNA from YEp24 (Botstein et al., supra) was cut with restriction enzymes HaeIII and HpaI and the DNA run on a preparative 1.0% Agarose gel. The 1.4 kb fragment containing the 2u origin of replication was identified by comparison with the migration pattern of molecular weight marker fragments and electroeluted into a well cut into the agarose. The DNA fragment was purified by passing the buffer from the well over a DEAE Sephacel column (Maniatis et al, supra). Plasmid YIp5 was cut with EcoRI and the "sticky ends" "filled in" using the Klenow fragment of DNA polymerase 1 and all four dNTP's. The HaeIII/HpaI fragment from YEp24 was ligated into the "filled in" EcoRI site of YIp5 (Fig. 7). The ligation mixture was transformed into HB101 and the resulting ampicillin resistant colonies screened for the presence of the 2u origin fragment. Since a "filled in" EcoRI site ligated to a HaeIII site re-creates the EcoRI site the orientation of the fragment was determined by mapping the resulting EcoRI site to restriction sites on the plasmid. A plasmid (YOpI) having the EcoRI site proximal to the PstI site within the ampicillin resistant gene was used in subsequent constructions.

## Example 1

## Loss of ATP by futile cycling

## Isolation of the gene for Fructose 1,6-diphosphatase

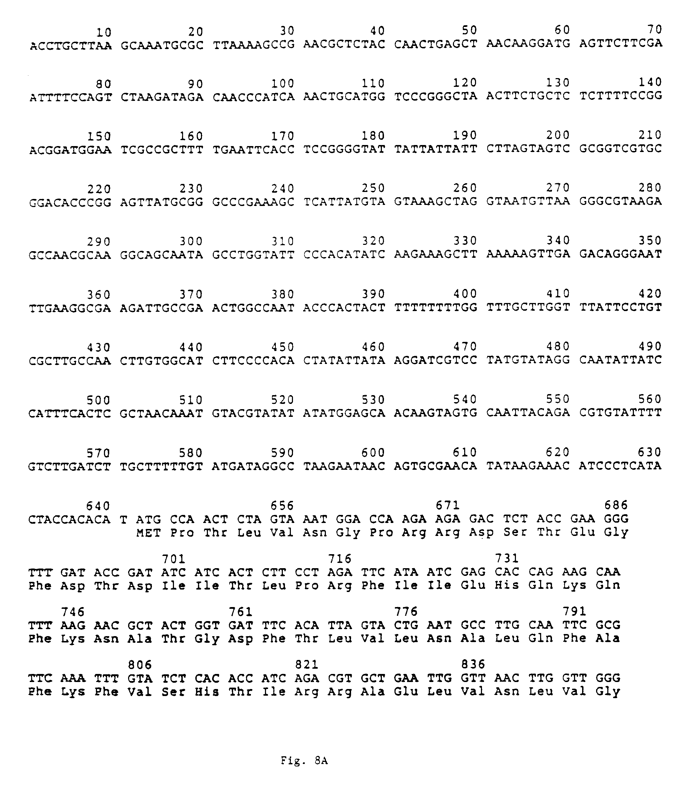

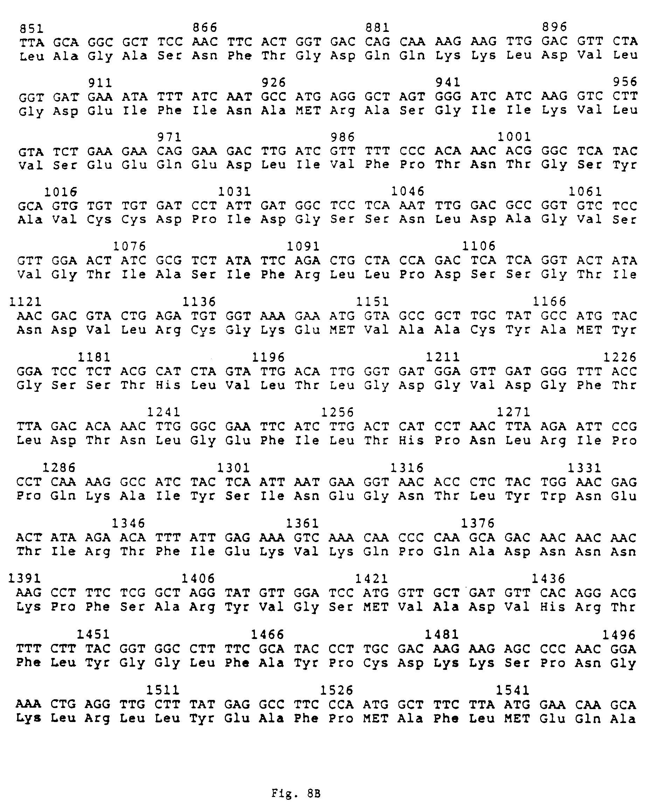

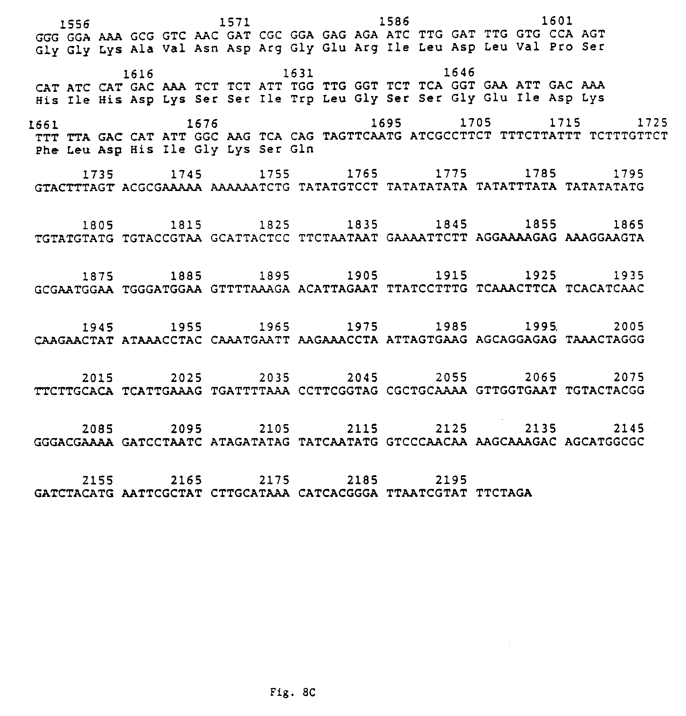

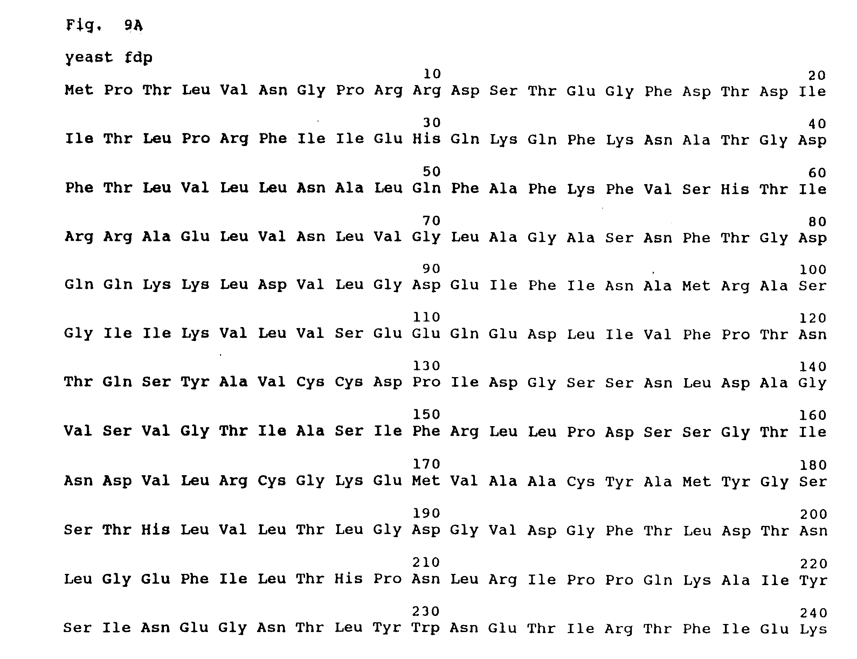

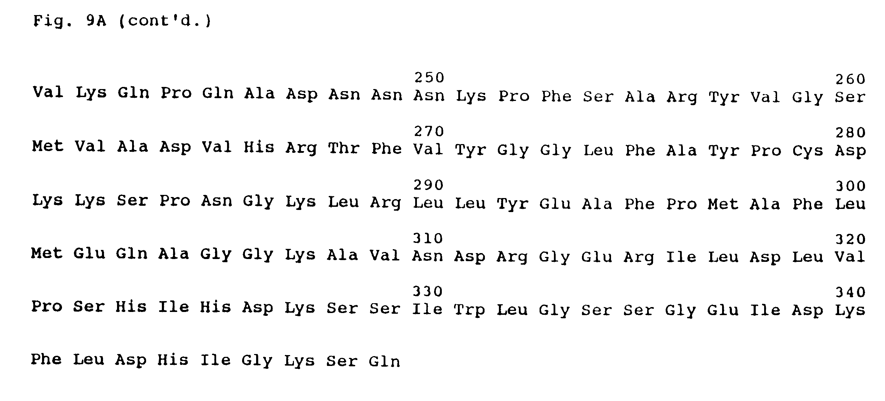

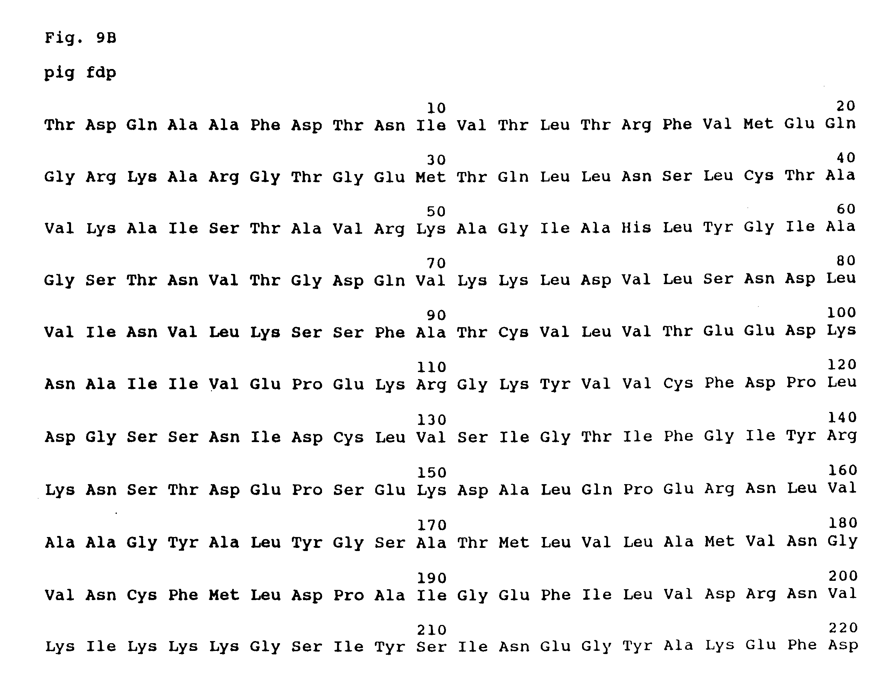

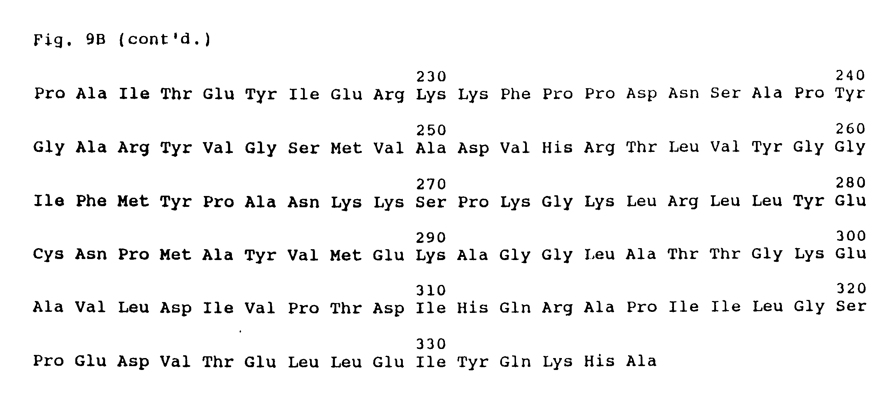

The gene for FDPase was isolated by complementation of a deletion mutant of FDPase in E. coli (strain DF657, CGSC number 6695). A plasmid library of "wild type" yeast genomic DNA in a pBR322 plasmid vector was transformed into DF657 by selection for antibiotic resistance and a plasmid carrying the yeast FDPase gene identified by its ability to allow the bacteria to grow on a gluconeogenic carbon source. The yeast FDPase clone was sequenced using the dideoxynucleotide sequencing method of Sanger et al., Proc. Natl. Acad. Sci. USA 74: 5463-5467, 1977. Fig. 8. Comparison of the amino acid sequence of yeast FDPase derived from the DNA sequence showed greater than 50% homology with the amino acid sequence of purified pig FDPase (Marcus et al., (1982) Proc. Natl. Acad. Sci. USA 79:7161-7165) (Fig. 9) confirming the correct identification of the yeast clone.

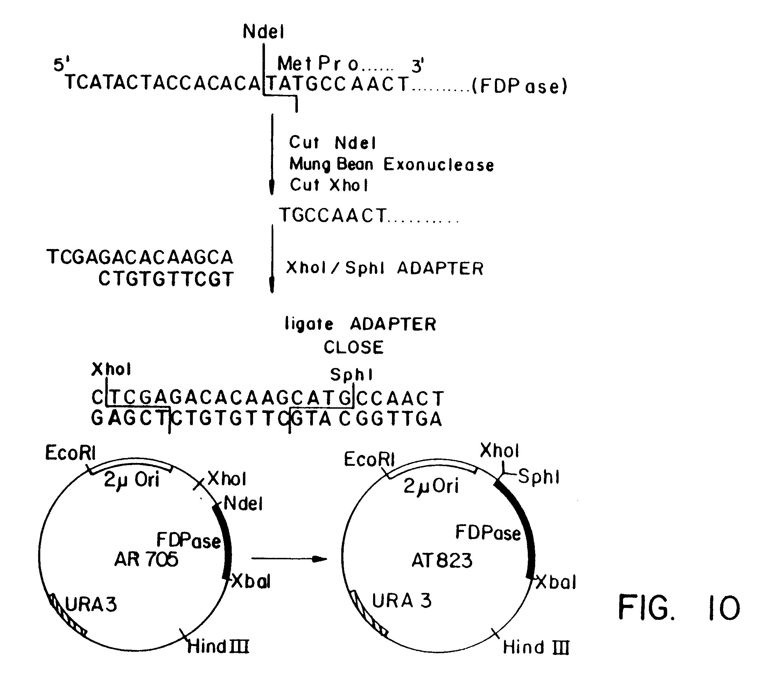

Since a futile cycle must be carefully regulated to prevent premature wastage of ATP the first adaption of the natural FDPase gene was to change its promoter for that of a sequence which could be regulated during the fermentation. A restriction site analysis of the DNA sequences of yeast FDPase identified a NdeI site very close to the start of the coding region (Fig. 10). In vitro mutagenesis was used to adapt the 5' end of the clone for expression from a heterologous yeast promoter by converting the NdeI site to an SphI site. The FDPase gene was first subcloned into pBR322 to create plasmid AR705, Fig. 10. Plasmid AR705 was digested with NdeI and treated with mung bean exonuclease. An adapter containing four out of six base pairs of an SphI site at one end and a XhoI overhang at the other end was ligated to the plasmid, digested with XhoI and the plasmid closed using T4 DNA ligase to generate plasmid AT823, Fig. 10. The ligation mix was transformed into bacteria and ampicillin resistant colonies screened for the presence of the SphI and XhoI sites.

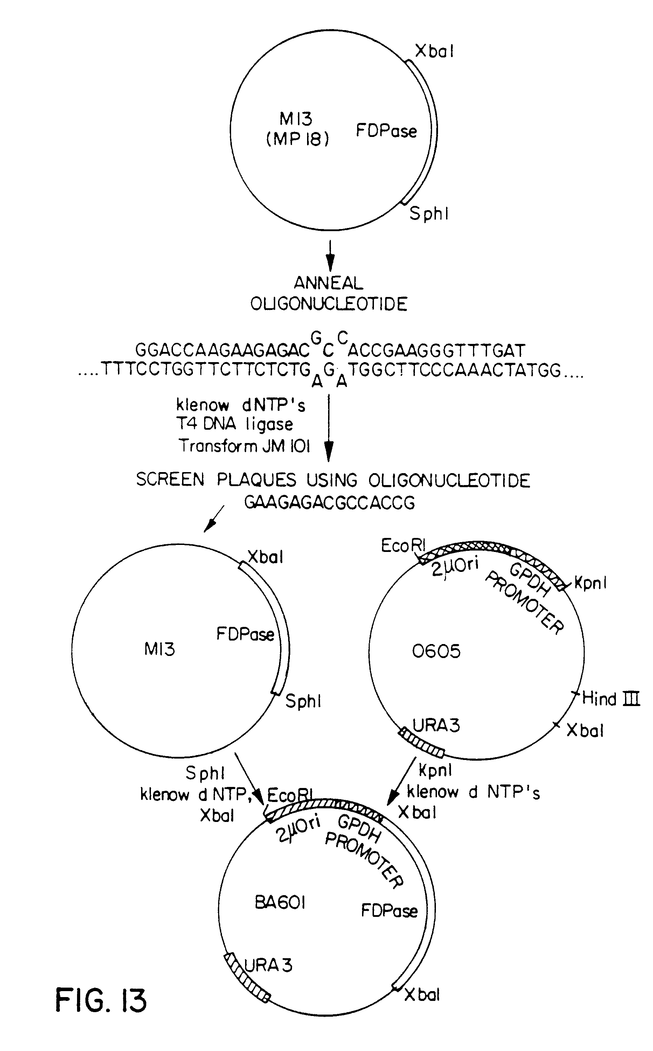

The FDPase gene in plasmid AT823 was ligated to the promoter from the gene for glyceraldehyde phosphate dehydrogenase (GAP491) [Holland and Holland (1980), J. Biol. Chem. 255:2596-2605] as follows. Plasmid O605 (Fig. 13), derived from plasmid M903, was digested with KpnI, treated with the Klenow fragment of DNA PolI, and cut with HindIII. Plasmid AT823 was cut with SphI, treated with the Klenow fragment of DNA PolI, cut with HindIII, and the 3.8 kb SphI/HindIII fragment was isolated and ligated to the HindIII/KpnI-digested O605 to generate plasmid AU125. Expression of the FDPase gene in plasmid AU125 is now regulated by the GPDH promoter.

FDPase mutagenesis

The plasmid containing the FDPase with the heterologous promoter was transformed into yeast and transformants tested for expression of the FDPase clone. FDPase activity was detectable when cells were grown under inducing conditions on a gluconeogenic or glycolytic carbon source. When growing using fermentation, it would be expected that allosteric inhibitors and enzyme inactivation would reduce enzyme activity.

Since inactivation is mediated by phosphorylation of a serine residue, a change in the structural gene contemplated by this invention is the elimination of this phosphorylation site. The serine residue which is phosphorylated has been identified as residue 12 (the only cAMP dependent protein kinase recognition site in the sequence and from peptide mapping of purified phosphorylated enzyme and amino acid sequencing of the phosphorylated peptide (Rittenhouse et al., (1986) J. Biol. Chem. 261:3939-3943).

It had previously been found that by performing a mild trypsin digest on liver FDPase one could generate an amino terminal deletion which retained activity (Chatterjee et al., 1985). Mammalian and yeast FDPase show a very high level of conservation. An amino terminal deletion of the yeast enzyme was made and tested for activity.

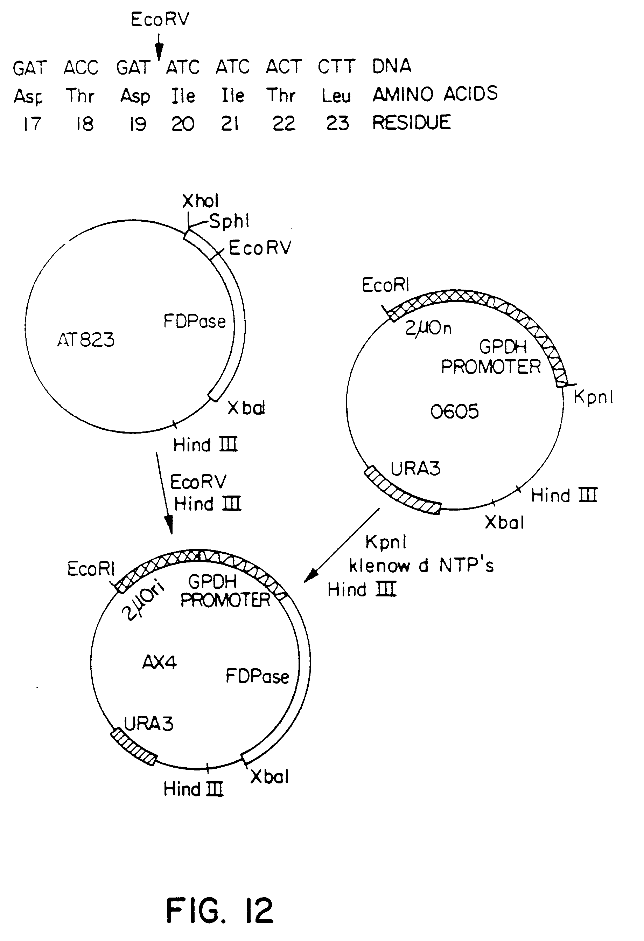

Fructose-1, 6-diphosphatase (FDPase) was hooked up to the yeast GPDH promoter from the EcoRV restriction site present in the DNA sequence within the amino terminus of the gene (Figure 12, AX4). This gave an amino-terminal deletion, to residue 19, which missed the protein kinase recognition site.

In addition, serine (residue 12) was changed to an alanine using site directed mutagenesis (Zoller and Smith, supra). This is a conservative change which would not be expected to affect the activity of the non-phosphorylated protein but would prevent phosphorylation of the enzyme. The serine could also have been changed to a threonine, valine or cysteine or another amino acid using site directed mutagenesis (Figure 13). This was achieved by cloning that part of the sequence around this residue into a single stranded DNA virus, M13. A synthetic oligonucleotide was made which hybridized to this region of the DNA but was a mismatch at the serine residue such that the sequence substitutes an alternate codon. A double stranded molecule was then made from this hybrid by the use of the Klenow fragment of DNA polymerase PolI. The reaction is conducted in the presence of all four deoxynucleotide triphosphates and DNA ligase. This hybrid double stranded DNA was then cloned into bacteria, replicated, re-isolated and re-transformed into bacteria to resolve the two strands. Half of the progeny contain the sequence for the serine, and half contain the substituted sequence for the alanine or other codon of choice. They were distinguished by hybridization to a short oligonucleotide (17 bp) complementary to the substituted sequence. This substituted gene was then put back into the multicopy GPDH expression vector as described above and transformed into a laboratory strain of yeast e.g. KY114.

In order to measure the rate of glycolysis, cultures of yeast expressing clones of FDPase from the glyceraldehyde phosphate dehydrogenase (GPDH) promoter were examined in small one liter fermentors. Cultures were grown in batch and nitrogen was bubbled through the culture at 110 ml/min. Carbon dioxide and cell density were measured periodically.

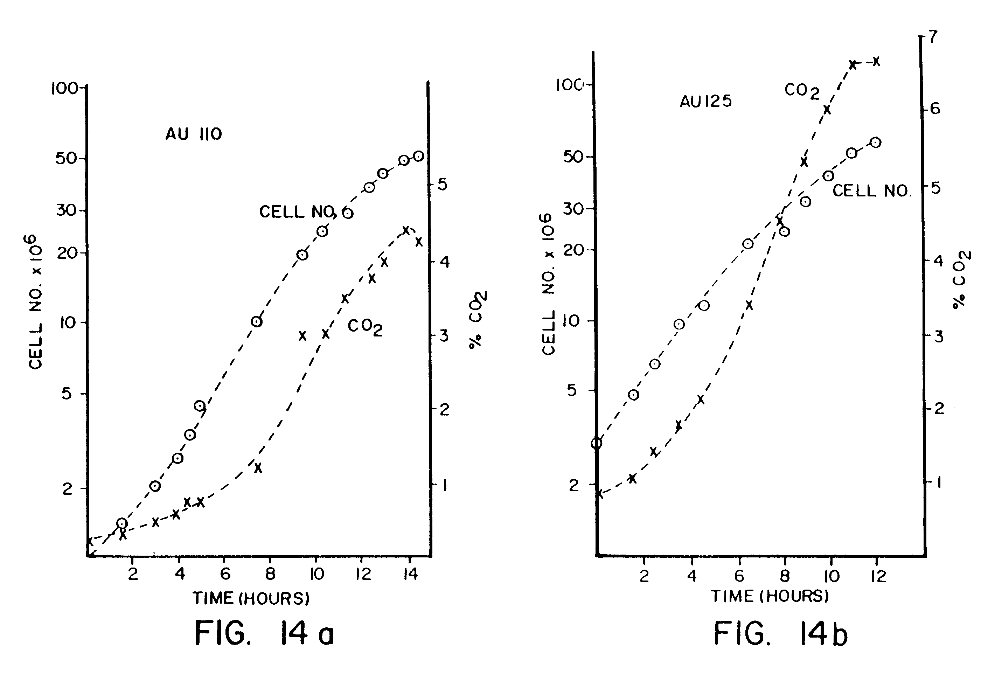

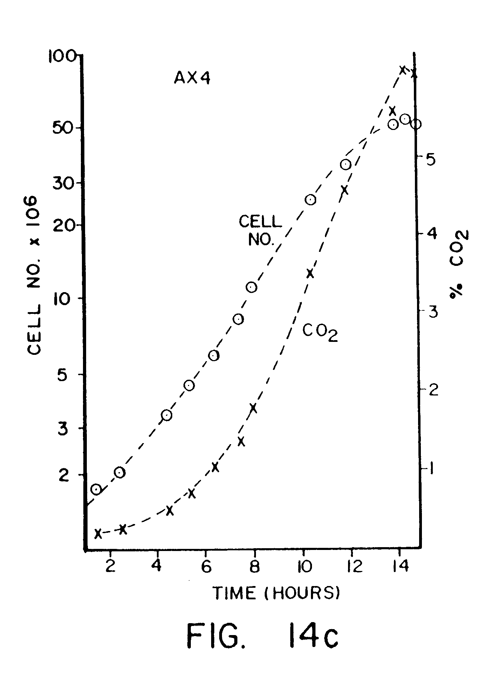

Initially, cultures of yeast expressing the wild type enzyme (plasmid AU125) and the amino terminal deletion (plasmid AX4) were compared to a control plasmid containing a non-expressed FDPase (plasmid AU110) (Figure 14). The level of carbon dioxide produced by the cultures at the beginning of the growth cycle is very similar. However at the end of the growth cycle, substantially higher levels of $CO_2$ were produced. The increase in carbon dioxide output of the strain carrying the amino terminal FDPase deletion is less than that expressing wild type enzyme. However FDPase does not appear to be inactivated in this yeast strain and the amino terminal deletion has a considerably lower specific activity than the "wild type" enzyme. One would therefor have expected that the wild type enzyme would give a greater stimulation of glycolysis than the enzyme containing the deletion. It is very intersting that carbon dioxide output increases only at the end of the growth cycle. This would suggest that FDPase is being regulated allosterically during exponential growth phase. The most likely candidates for this regulation are fructose-2, 6-diphosphate or AMP.

After specific point mutants in the phosphorylation site of FDPase had been created, fermentation experiments were repeated. In these experiments, strain ATCC 26675, expressing FDPase containing the serine to alanine mutation from the GPDH promoter (plasmid BA601) or a control plasmid where FDPase was not expressed (plasmid BA802) were tested. Strain ATCC 26675 has been found to give the highest level of inactivation of many yeast strains tested. These cultures should thus give a conservative estimate of the possible increase in gassing power.

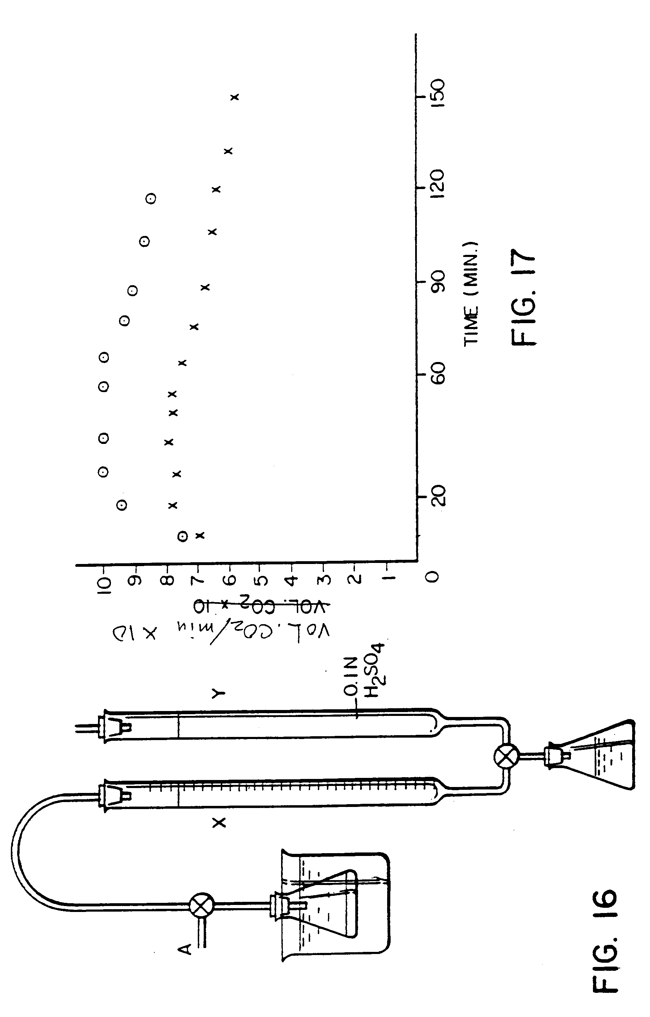

Fermentations were performed as before and carbon dioxide and cell density measured. Carbon dioxide output is again increased toward the end of the growth cycle in the strain expressing FDPase (Figure 16).

The fermentation experiment was repeated and gave good reproducibitity. Cultures were harvested at the end of the growth cycle, centrifuged, and examined using a gassing test described below.

Gassing tests were performed in an apparatus illustrated in Figure 16. The flask contained one gram of cells, one gram of glucose and 10ml of media (yeast nitrogen base, Difco) and had a final OD 600 nm of 12. All solutions were at 32°C. The water bath was also at 32°C. Tube a was open during the first 10 minutes after the flask was placed in the water bath. Measurements of $CO_2$ evolution were made periodically by closing tube a and measuring the amount of $CO_2$ evolved in burette X after adjusting the level of liquid in burette Y to that in burette X to bring the gas in burrette X to atmospheric pressure. Measurements were taken until 100 ml of $CO_2$ had been produced. The rate of $CO_2$ production in the strain containing the plasmids described above are illustrated in Figure 17. In this test the strains expressing the mutant FDPase gave an increase of 25% in gassing power.

In addition to the previously described modifications, this invention further contemplates alteration of the allosteric regulation of FDPase by fructose-2,6-diphosphate or AMP.

Fructose-2,6-phosphate is synthesized via an enzymatic pathway from fructose-6-phosphate by the enzyme fructose-6-phosphate-2-kinase (Clifton and Fraenkel, J. Biol. Chem., 258:9245 (1983) and Pilkis et al., J. Biol. Chem., 259:949 (1949)).

One method for reducing inhibition of enzyme activity is to mutate the cloned copy of FDPase in vitro - (see e.g., Shortle et al., Proc. Natl. Acad. Sci., 79:1588 (1982)) and introduce it back into the cell on a self-replicating selectable yeast plasmid followed by assaying for the loss of the inhibitory effects of fructose-2,6-diphosphate and AMP. In principal, the loss of a site where an allosteric inhibitor binds is often a fairly conservative change in the enzyme structure since even a slight modification of the binding site is expected to greatly alter its affinity for fructose-2,6-diphosphate. This approach requires a food assay for the altered enzyme. Since FDPase is under the control of an inducible promoter, when the futile cycle is working efficiently, under inducing conditions, mutant colonies growing on a fermentable carbon source are very small but under non-inducing conditions the colonies are normal in size. The suspected mutant colonies are also plated on a gluconeogenic carbon source where they grow normally under inducing conditions. Such colony screening methods may therefore be used to assay for the altered enzyme.

Finally, the altered FDPase is introduced into the strain of yeast used for baking by the procedures described above. Bakers yeast containing the altered FDPase are found to have substantially increased leavening ability.

Example 2

Expression of a cytoplasmic acid phosphatase

Preparation of the Promoter Fragment of APase

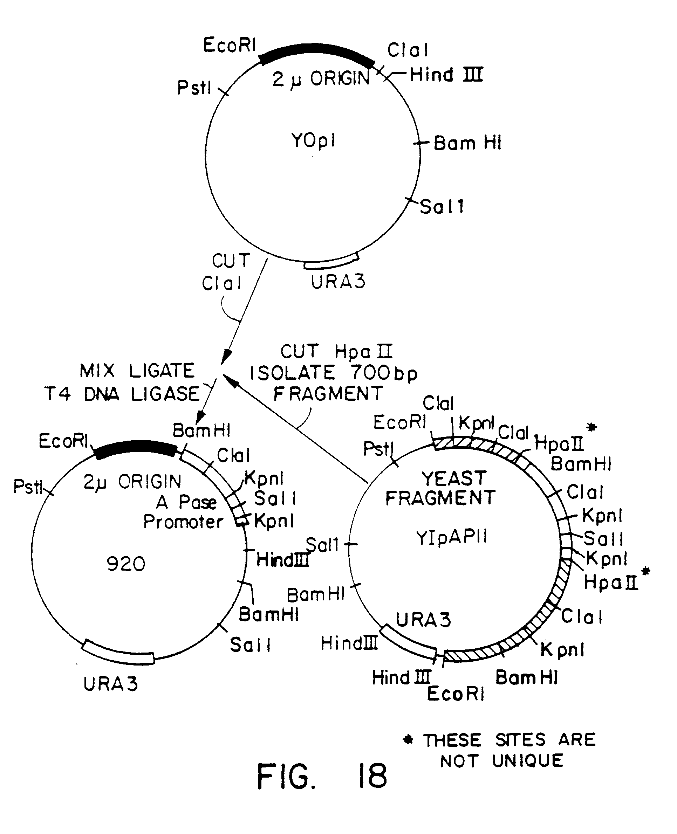

Plasmid YIpAPII (Rogers et al., 1982, supra) containing a full copy of the large subunit, P60 (PHO 5), of the acid phosphatase enzyme was mapped with various restriction enzymes and HpaII was found to give a 700 bp restriction fragment containing 600 bp of upstream DNA sequence from the initiator (or start) codon for the structural gene whose position is known by reference to the ClaI site on the fragment (Thill et al., Molecular Cell Biology 3, pp. 570-9 (1983)). Plasmid YIpAPII was cut with HpaII and the DNA run on a preparative 1.5% agarose gel. The band of 700 bp, containing the promoter was electroeluted into a well cut into the gel as before and purifed on a DEAE Sephacel column. The fragment was mixed with YOpI cut with ClaI and the DNA ligated with T4 DNA ligase. Since HpaII and ClaI have self complementary "sticky ends" these DNA's will ligate together. The ligation mix was transformed into HB101 and the ampicillin resistent colonies screened for the presence of the promoter fragment. One such plasmid, 920, (see Fig.18) was used for further constructions.

From the DNA sequence of the fragment of the acid phosphatase (PHO 5) gene, Fig. 3 (Thill et al., 1983, supra; Arima et al., N.A.R. 11, pp. 1657-72 (1983), an area having four out of the six bases of a BglII restriction endonuclease recognition site was identified 5bp upstream from the initiator ATG. This area was used to create a BglII site at this point in the APase promoter sequence using a synthetic oligonucleotide linker of self complementary sequence CTAGCATGCTAG.

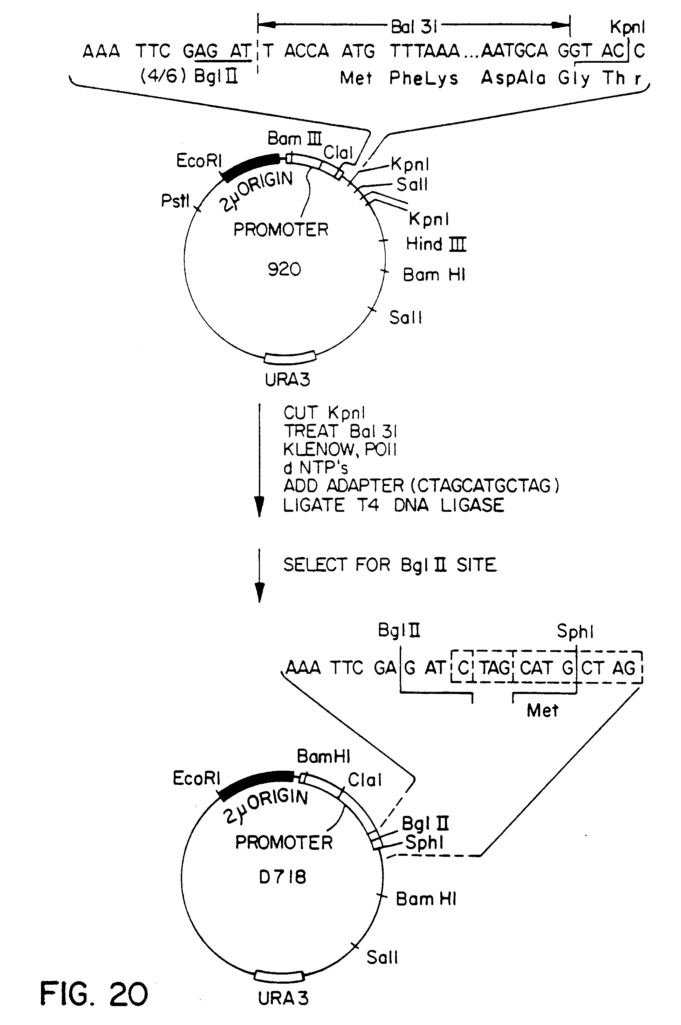

Plasmid 920 was cut with KpnI (see Fig. 20) and the DNA treated with the double strand exonuclease Bal31 (Legerski et al., N.A.R. 5, pp.1145-1463 (1978)). At set time intervals, the reaction was stopped by the addition of ethylenediaminetetraacetic acid (EDTA) to 0.05M (Legerski et al., (1978) supra). A portion of the plasmid from each time interval was digested with ClaI and run out on a 12% poly-acrylamide gel. The time point where the 300 bp ClaI/KpnI fragment had been digested to approximately 270 bp was noted. The remainder of the Bal31 treated plasmid from this time point was treated with the Klenow fragment of DNA Polymerase I and the four dNTP's. A linker of self complementary sequence CTAGCATGCTAG was kinased, annealed and ligated to this plasmid DNA. The DNA was then circularized with T4 DNA ligase and transformed into HB101. Approximately two thousand ampicillin resistant colonies were washed from the plates and supercoiled plasmid DNA made from these E. coli cells (Maniatis et al, 1982, supra). This pooled plasmid DNA was cut with the restriction enzyme BglII and the DNA run out on a preparative agarose gel. DNA running as a cut linear band was eluted into a well cut into the agarose and purified on a DEAE sephacel column. This purified DNA was re-circularized with T4 DNA ligase and transformed into HB101. Ampicillin resistant colonies were screened for the presence of a BglII site. The only way for the plasmid to obtain a BglII site was for the site to be created at the junction of the Bal31 digested DNA and the linker. The only available sequence where this could occur within several hundred bp upstream of the KpnI site is 5b in front of the ATG initiation codon. One such plasmid containing a BglII site, D718 (Fig. 20) was checked and shown to be as expected by the dideoxynucleotide sequencing method of Sanger (Sanger et al, Proc. Natl. Acad. Sci. 74, pp. 5463-67 (1977)).

Plasmid YIpAP11, has been deposited with the American Type Culture Collection in E. coli HB101 as follows:

E. coli HB101 (YIpAP11) - ATCC No. 39570

### Creation of a restriction site at the leader/native protein junction

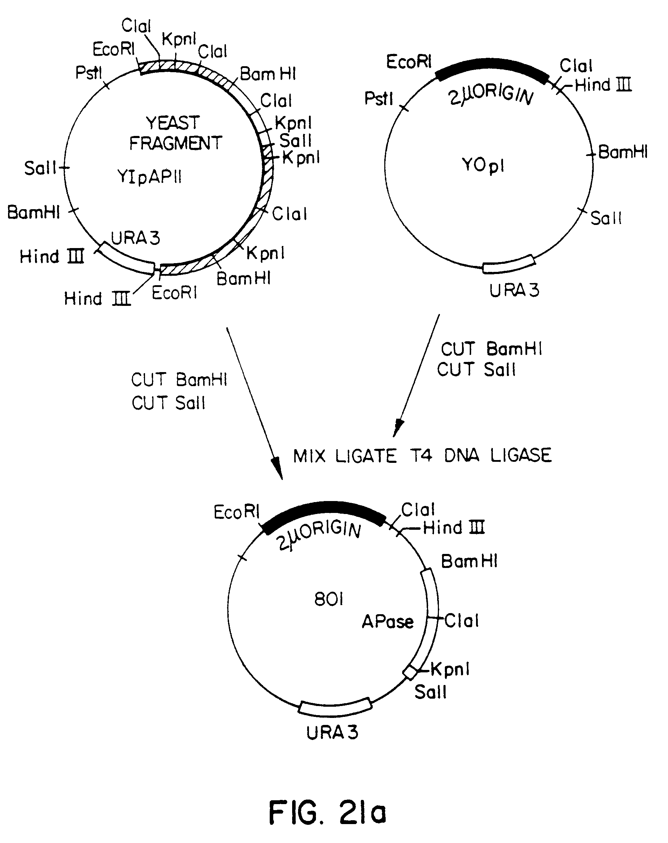

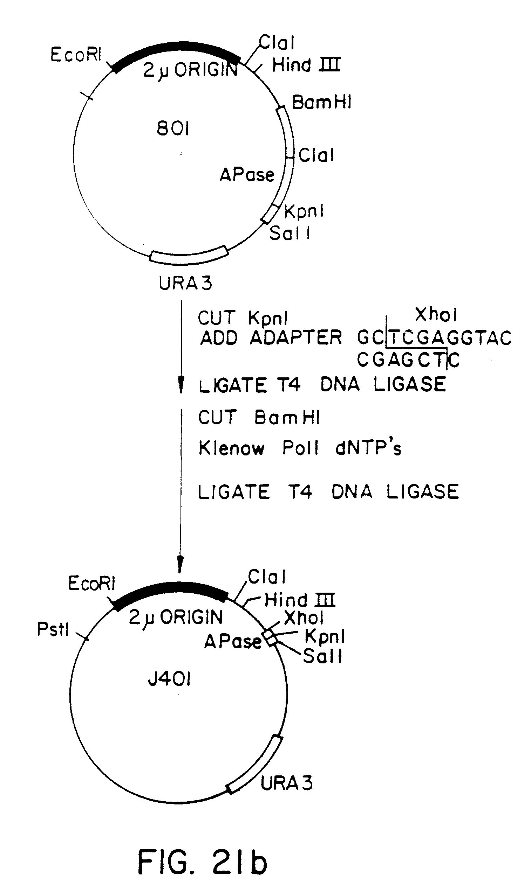

From the sequence of acid phosphatase gene it can be seen (Fig. 18) that there is a KpnI site close to the start of the mature sequence. This has enabled us to introduce a restriction site at the junction of the leader sequence and the mature protein sequence GCTCGAGGTAC using a synthetic oligonucleotide of sequence CGAGCTC. Since there are several KpnI sites in the acid phosphatase gene a fragment had to be subcloned from the 5' end of the gene. Plasmid YIpAP11 was cut with BamHI and SalI and the fragment containing the promoter subcloned into the BamHI/SalI sites of YOp1. (Fig. 21) Transformed bacteria were screened and plasmid 801 was found to have the correct sequence. To introduce a restriction site at the 5' end of the mature sequence plasmid 801 was cut with KpnI and the adapter described above ligated to the KpnI site (Fig 21). The plasmid was then cut with BamHI and the site "filled in" with the Klenow fragment of DNA polymerase I and the plasmid re-circularized. Transformed bacterial colonies were screened for the presence of the adapter. One such plasmid (J401) was used for further constructions. As can be seen from Fig 21 the adapter creates a XhoI site at the junction of the leader and mature acid phosphatase such that if the plasmid is cut with XhoI and the overhang digested with mung bean exonuclease there is a blunt site created at the correct position in the sequence at the start of the mature coding region.

### Synthesis of a "leader less" clone of acid phosphatase

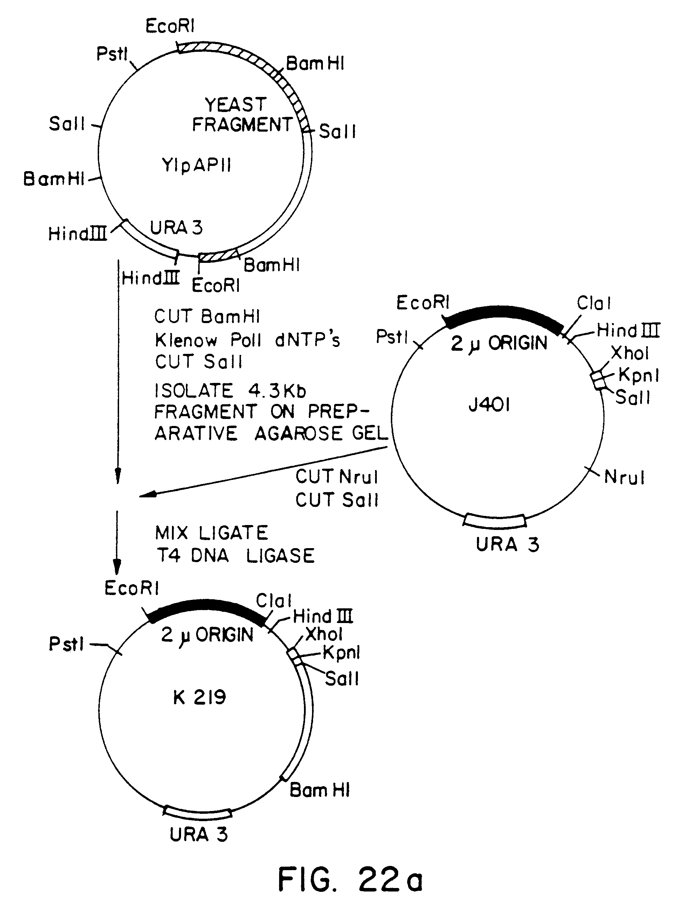

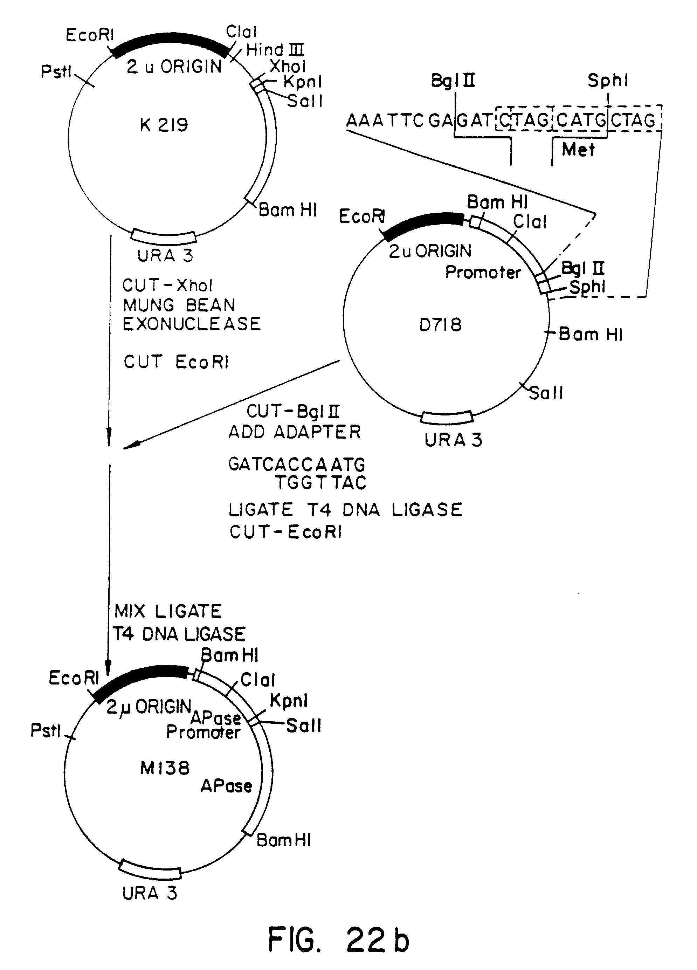

First the full length copy of acid phosphatase was constructed from plasmid J401. Plasmid YIpAP11 was cut with BamHI, the 5' overhang "filled in" with the Klenow fragment of DNA polymerase I and then cut with SalI and the BamHI/SalI fragment containing the APase gene purified by preparative gel electrophoresis. This fragment was then ligated into the SalI/NruI sites of plasmid J401 (Fig.22). A "filled in" BamHI site ligated to an NruI site recreates the BamHI site. Next the acid phosphatase promoter was reattached to the structural gene. Plasmid D718 was cut with BglII and an adapter of sequence GATCACCAATG which recreates the acid phosphatase TGGTTAC promoter sequence to the initiator methionine codon, ligated to the BglII site. The plasmid was then cut with EcoRI and the EcoRI to BglII adapter fragment (Fig 22) cloned into plasmid K219 which had been cut with XhoI, treated with mung bean exonuclease to flush the ends of the DNA and then cut with EcoRI. Transformants were screened and plasmids containing the correct restriction fragments were sequenced using the dideoxynucleotide sequencing method. Plasmid M138 was found to have the correct sequence at the junction of the promoter and the mature gene.

### Addition of a yeast centromere to the plasmid

The acid phosphatase promoter is inducible about 1,000 fold. The copy number of a yeast plasmid may be varied by using different origins of replication or a yeast centromere (Clark & Carbon, 1980; Tschumper & Carbon, 1983). A yeast centromere lowers the copy number of a 2u origin plasmid to about 1 copy per cell.

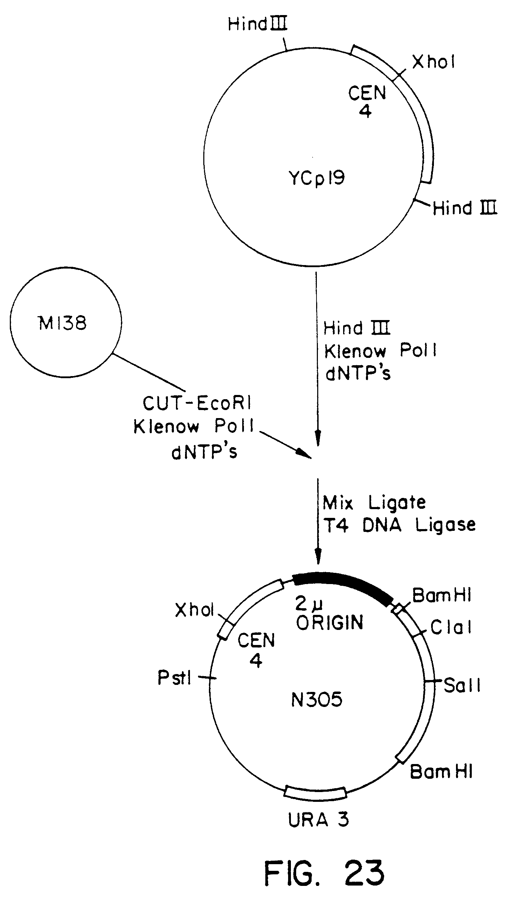

Plasmid M138 was cut with EcoRI and the ends blunted with the Klenow fragment of DNA polymerase I. Next, plasmid YCP19 (Fig. 23), which contains the centromere from chromosome 4 of yeast (Parent et al., supra), was cut with HindIII and the ends blunted with the Klenow fragment of DNA polymerase I. The fragment containing the centromere was then ligated into the EcoRI site of M138 to produce plasmid N305 (Fig 22).

Expression of cytoplasmic acid phosphatase

Plasmids M138 and N305 were transformed into yeast together with a control plasmid M721. Uracil prototrophs were selected and grown in high phosphate MO media containing an excess of glucose(4%), in a 1 liter New Brunswick Scientific model F-200 fermenter. During growth, cell density was measured using a Bausch and Lomb Model Spectronic 20 at 600 nm. The results are set forth in Tables 1 and 2 below. The fermenter was run at 30°C and an agitation setting of 4. Nitrogen was continuously bubbled through the vessel at a rate of 430 cc/min and the off gas passed through a moisture trap of Dry-Rite and into a Perkin Elmer Mass Spectrometer gas analyzer to measure $CO_2$.

## TABLE 2

## CARBON DIOXIDE EVOLUTION IN UNINDUCED CULTURES

## CONTROL PLASMID M721

| Time Minutes | Cell Number $(x10^6)$ | % Carbon Dioxide |
|---|---|---|
| 0 | 0.25 | 0.08 |
| 80 | 0.64 | 0.21 |
| 150 | 0.90 | 0.16 |
| 215 | 1.35 | 0.19 |
| 280 | 1.8 | 0.26 |
| 340 | 2.3 | 0.34 |
| 405 | 3.3 | 0.45 |
| 470 | 4.75 | 0.61 |
| 520 | 6.25 | 0.73 |
| 580 | 7.0 | 0.79 |
| 630 | 8.5 | 0.85 |
| 690 | 10.0 | 0.85 |
| 710 | 11.0 | 0.75 |

## TABLE 2 CON'T

### PLASMID N305

| Time Minutes | Cell Number ($x10^6$) | % Carbon Dioxide |
|---|---|---|
| 0 | 1.1 | 0.17 |
| 60 | 1.35 | 0.22 |
| 108 | 1.5 | 0.26 |
| 165 | 2.0 | 0.35 |
| 245 | 3.0 | 0.50 |
| 345 | 5.0 | 0.76 |
| 390 | 6.5 | 0.92 |
| 465 | 8.5 | 1.05 |
| 520 | 10.0 | 1.15 |
| 610 | 14.5 | 1.02 |
| 685 | 16.0 | 0.86 |

## TABLE 2 CON'T

### PLASMID M138

| Time Minutes | Cell Number ($x10^6$) | % Carbon Dioxide |
|---|---|---|
| 0 | 2.7 | 0.72 |
| 48 | 3.5 | 0.77 |
| 77 | 4.0 | 0.87 |
| 107 | 5.0 | 1.02 |
| 165 | 7.2 | 1.31 |
| 200 | 8.0 | 1.56 |
| 238 | 8.5 | 1.70 |
| 286 | 10.5 | 1.75 |
| 363 | 13.0 | 1.75 |
| 450 | 16.5 | 1.65 |
| 568 | 20.0 | 1.60 |
| 632 | 21.0 | 1.44 |

The level of carbon dioxide produced by the strains carrying the three different plasmids during growth on high phosphate media was found to vary (Table 1). Then the basal level of promoter activity in high phosphate media is sufficient to produce an effect on the rate of glycolysis. When the data was compiled and normalized for the same stage in the growth cycle, it was noted that the level of carbon dioxide produced by cultures growing in high phosphate media increased with the copy number of the plasmid. The strain carrying the multicopy plasmid produced twice as much carbon dioxide as the control and the strain carrying the single copy plasmid produced an intermediate level. Thus, the level of acid phosphatase can be controlled thereby controlling the level of cytoplasmic ATP in accord with this invention and increasing the rate of production of carbon dioxide.

Example 3

The effect of Plasma Membrane Uncouplers on the Rate of Glycolysis

When increasing amounts 2,4-dinitrophenol were added to stable chemostat cultures of Fleishman's baking yeast a considerable stimulation in carbon dioxide production is observed.

Initial experiments were performed to determine the basal level of carbon dioxide per cell under the growth conditions defined by the chemostat and to determine the stability of the culture. An overnight culture was inoculated into the chemostat and grown up for twenty-four hours as a batch culture. Media feed was started and the culture left to stabilize for a further twenty-four hours before measurements were taken. Carbon dioxide production and cell number were monitored over a forty-eight hour period. These results are shown in Table 3. Time is given in hours after media addition start-up. Carbon dioxide is given as a percentage in the gas stream.

Table 3

| Chemostat Stability | | | |
|---|---|---|---|
| Time after startup Hours | Culture Density O.D. 600 nm | Cell Number Cells/ml x $10^{-8}$ | Carbon Dioxide in the off gas % |
| 26 | 0.33 | 1.1 | 2.4 |
| 29.5 | 0.32 | 1.1 | 2.3 |
| 32 | 0.31 | 1.0 | 2.3 |
| 34 | 0.33 | 1.1 | 2.2 |
| 50 | 0.31 | 1.0 | 2.3 |

The culture appeared to be relatively stable.

Dinitrophenol was added to the media feed at 5 uM. Dinitrophenol was also added to the culture vessel at 5 uM as given in Experimental Procedures.

The culture was left to stabilize for forty-eight hours. As can be seen from Table 4, 5 uM dinitrophenol had little effect on carbon dioxide production.

Table 4

| The Effect of Dinitrophenol On The Rate of Fermentation | | | |
|---|---|---|---|
| Concentration of Dinitrophenol uM | Culture Density cells/ml x $10^{-8}$ | Carbon Dioxide in the off gas % | Relative Carbon Dioxide Production per cell |
| 0 | 1.1 | 2.2 | 1.0 |
| 5 | 1.1 | 2.3 | 1.0 |
| 20 | 0.75 | 1.85 | 1.2 |
| 50 | 0.75 | 2.0 | 1.3 |
| 100 | 0.67 | 2.0 | 1.5 |
| 200 | 0.55 | 1.9 | 1.7 |
| 0 | 1.1 | 2.3 | 1.0 |

The concentration of dinitrophenol in the culture was slowly increased stepwise to 200 uM. After each step addition of dinitrophenol, the culture was left to stabilize for thirty-six to forty-eight hours before measuring cell density and carbon dioxide concentration. As shown in Table 4, above, with increasing concentration of dinitrophenol there was an increase in the level of carbon dioxide produced per cell.

If the effect of dinitrophenol on cellular metabolism had been the uncoupling of the plasma membrane ATPase one would have expected an increase in carbon dioxide output and a corresponding decrease in the cell density. It should be remembered that the dissipation of the plasma membrane ion gradient would also affect other cellular processes dependent on this ion gradient i.e transport. It would not, therefore, be surprising to find that the metabolic efficiency of the cell had been reduced by this treatment. This level of stimulation of glycolysis would therefore be expected to be a minimum.

The experiments have demonstrated that carbon dioxide production can be stimulated in a dose dependent way by dinitrophenol. The level of stimulation was found to be highly reproducible and gave a greater than two-fold increase in carbon dioxide production at the highest concentration used.

**Claims**

1. A vector DNA containing a gene encoding an enzyme under the expression control of a promoter, wherein said gene encodes the enzyme fructose-1,6-diphosphatase inducible in the presence of glucose by activating said promoter.

2. The vector DNA of claim 1, wherein the gene encoding the enzyme fructose-1,6-diphosphatase is genetically modified such that codon 12 thereof encodes alanine, threonine, valine or cysteine, and/or a binding site for an allosteric inhibition of said enzyme is removed.

3. The vector DNA of claim 1 or 2, wherein the promoter is a regulatable or a constitutive promoter.

4. The vector DNA of claim 3, wherein the regulatable promoter is the galactose promoter, the maltose promoter, the phosphate metabolism promoter, the nitrogen metabolism promoter, the isocytochrome promoter, the alcohol dehydrogenase promoter, or a temperature sensitive promoter.

5. A process for increasing the rate of carbon dioxide and ethanol production in yeast by transforming the yeast with a vector of any one of claims 1 to 4.

6. A yeast cell containing a vector of any one of claims 1 to 4.

7. Use of a yeast of claim 6 for leavening, brewing or fermentation.

**Patentansprüche**

1. Vektor-DNA, enthaltend ein Gen, das ein Enzym unter der Expressionskontrolle eines Promotors codiert, wobei das Gen das Enzym Fructose-1,6-diphosphatase codiert, das in Gegenwart von Glucose durch die Aktivierung des Promotors induzierbar ist.

2. Vektor-DNA nach Anspruch 1, wobei das das Enzym Fructose-1,6-diphosphatase codierende Gen genetisch so modifiziert ist, daß dessen Codon 12 Alanin, Threonin, Valin oder Cystein codiert und/oder eine Bindungsstelle für eine allosterische Hemmung des Enzyms entfernt ist.

3. Vektor-DNA nach Anspruch 1 oder 2, wobei der Promotor ein regulierbarer oder ein konstitutiver Promotor ist.

4. Vektor-DNA nach Anspruch 3, wobei der regulierbare Promotor der Galactose-Promotor, der Maltose-Promotor, der Phosphat-Metabolismus-Promotor, der Stickstoff-Metabolismus-Promotor, der Isocytochrom-Promotor, der AlkoholDehydrogenase-Promotor oder ein temperaturempfindlicher Promotor ist.

5. Verfahren zur Steigerung der Herstellungsrate für Kohlendioxid und Ethanol in Hefe durch Transformation der Hefe mit einem Vektor nach einem der Ansprüche 1 bis 4.

6. Hefezelle enthaltend einen Vektor nach einem der Ansprüche 1 bis 4.

7. Verwendung einer Hefe nach Anspruch 6 zur Teigsäuerung, zum Brauen oder zur Gärung.

**Revendications**

1. Vecteur d'ADN contenant le gène codant pour une enzyme sous le contrôle de l'expression d'un promoteur où ledit gène code pour la fructose -1,6-diphosphatase inductible en présence de glucose en activant ledit promoteur.

2. Vecteur ADN selon la revendication 1, caractérisé en ce que le gène codant pour l'enzyme fructose-1,6-diphosphatase est modifié génétiquement de sorte que le codon 12 code pour l'alanine, la théronine, la valine ou la cystéine et/ou un site de liaison de l'inhibition allostérique de ladite enzyme est éliminé.

3. Vecteur ADN selon l'une des revendications 1 ou 2, où le promoteur est un promoteur régulable ou un promoteur constitutif.

4. Vecteur ADN selon la revendication 3, où le promoteur régulable est le promoteur du galactose, le promoteur du maltose, le promoteur du métabolisme du phosphate, le promoteur du métabolisme de l'azote, le promoteur isocytochrome, le promoteur de l'alcool déshydrogénase ou un promoteur sensible à la température.

5.  Procédé pour augmenter le taux de production de dioxyde de carbone et d'éthanol par transformation de la levure par un vecteur de l'une quelconque revendication 1 à 4.

6.  Cellule de levure contenant un vecteur selon l'une quelconque des revendications 1 à 4.

7.  Utilisaiton de la levure selon la revendication 6 pour la levée des pâtes, pour le brassage de la bière ou pour la fermentation.

FIG. 1

FIG. 2

24

FIG. 3

GLUCOSE MEDIUM

GAL PROMOTER          FLP RECOMBINASE

HETEROLOGOUS GENE

RECOMBINATION                    RECOMBINATION
SITE                                  SITE

# FIG. 4A

GALACTOSE  MEDIUM

GAL PROMOTER          FLP  RECOMBINASE

EXPRESSION

RECOMBINATION

LOST

# FIG. 4B

GLUCOSE MEDIUM

GAL PROMOTER    FLP RECOMBINASE

GPDH
UAS              BLOCK              FDPase

RECOMBINATION    RECOMBINATION
SITE             SITE

# FIG. 5A

GALACTOSE MEDIUM

GAL PROMOTER         FLP

EXPRESSION

BLOCK

RECOMBINATION

FDPase

UAS

BLOCK

LOST

GPDH
UAS              FDPase

EXPRESSION

# FIG. 5B

FIG. 6

FIG. 7

```
         10         20         30         40         50         60         70
ACCTGCTTAA GCAAATGCGC TTAAAAGCCG AACGCTCTAC CAACTGAGCT AACAAGGATG AGTTCTTCGA


         80         90        100        110        120        130        140
ATTTTCCAGT CTAAGATAGA CAACCCATCA AACTGCATGG TCCCGGGCTA ACTTCTGCTC TCTTTTCCGG


        150        160        170        180        190        200        210
ACGGATGGAA TCGCCGCTTT TGAATTCACC TCCGGGGTAT TATTATTATT CTTAGTAGTC GCGGTCGTGC


        220        230        240        250        260        270        280
GGACACCCGG AGTTATGCGG GCCCGAAAGC TCATTATGTA GTAAAGCTAG GTAATGTTAA GGGCGTAAGA


        290        300        310        320        330        340        350
GCCAACGCAA GGCAGCAATA GCCTGGTATT CCCACATATC AAGAAAGCTT AAAAAGTTGA GACAGGGAAT


        360        370        380        390        400        410        420
TTGAAGGCGA AGATTGCCGA ACTGGCCAAT ACCCACTACT TTTTTTTTGG TTTGCTTGGT TTATTCCTGT


        430        440        450        460        470        480        490
CGCTTGCCAA CTTGTGGCAT CTTCCCCACA CTATATTATA AGGATCGTCC TATGTATAGG CAATATTATC


        500        510        520        530        540        550        560
CATTTCACTC GCTAACAAAT GTACGTATAT ATATGGAGCA ACAAGTAGTG CAATTACAGA CGTGTATTTT


        570        580        590        600        610        620        630
GTCTTGATCT TGCTTTTTGT ATGATAGGCC TAAGAATAAC AGTGCGAACA TATAAGAAAC ATCCCTCATA


        640                   656                   671                   686
CTACCACACA T ATG CCA ACT CTA GTA AAT GGA CCA AGA AGA GAC TCT ACC GAA GGG
             MET Pro Thr Leu Val Asn Gly Pro Arg Arg Asp Ser Thr Glu Gly

              701                   716                   731
TTT GAT ACC GAT ATC ATC ACT CTT CCT AGA TTC ATA ATC GAG CAC CAG AAG CAA
Phe Asp Thr Asp Ile Ile Thr Leu Pro Arg Phe Ile Ile Glu His Gln Lys Gln

746                   761                   776                   791
TTT AAG AAC GCT ACT GGT GAT TTC ACA TTA GTA CTG AAT GCC TTG CAA TTC GCG
Phe Lys Asn Ala Thr Gly Asp Phe Thr Leu Val Leu Asn Ala Leu Gln Phe Ala

    806                   821                   836
TTC AAA TTT GTA TCT CAC ACC ATC AGA CGT GCT GAA TTG GTT AAC TTG GTT GGG
Phe Lys Phe Val Ser His Thr Ile Arg Arg Ala Glu Leu Val Asn Leu Val Gly
```

Fig. 8A

```
851                  866                  881                  896
TTA GCA GGC GCT TCC AAC TTC ACT GGT GAC CAG CAA AAG AAG TTG GAC GTT CTA
Leu Ala Gly Ala Ser Asn Phe Thr Gly Asp Gln Gln Lys Lys Leu Asp Val Leu

       911                  926                  941                  956
GGT GAT GAA ATA TTT ATC AAT GCC ATG AGG GCT AGT GGG ATC ATC AAG GTC CTT
Gly Asp Glu Ile Phe Ile Asn Ala MET Arg Ala Ser Gly Ile Ile Lys Val Leu

            971                  986                  1001
GTA TCT GAA GAA CAG GAA GAC TTG ATC GTT TTT CCC ACA AAC ACG GGC TCA TAC
Val Ser Glu Glu Gln Glu Asp Leu Ile Val Phe Pro Thr Asn Thr Gly Ser Tyr

   1016                 1031                 1046                 1061
GCA GTG TGT TGT GAT CCT ATT GAT GGC TCC TCA AAT TTG GAC GCC GGT GTC TCC
Ala Val Cys Cys Asp Pro Ile Asp Gly Ser Ser Asn Leu Asp Ala Gly Val Ser

            1076                 1091                 1106
GTT GGA ACT ATC GCG TCT ATA TTC AGA CTG CTA CCA GAC TCA TCA GGT ACT ATA
Val Gly Thr Ile Ala Ser Ile Phe Arg Leu Leu Pro Asp Ser Ser Gly Thr Ile

1121                 1136                 1151                 1166
AAC GAC GTA CTG AGA TGT GGT AAA GAA ATG GTA GCC GCT TGC TAT GCC ATG TAC
Asn Asp Val Leu Arg Cys Gly Lys Glu MET Val Ala Ala Cys Tyr Ala MET Tyr

       1181                 1196                 1211                 1226
GGA TCC TCT ACG CAT CTA GTA TTG ACA TTG GGT GAT GGA GTT GAT GGG TTT ACC
Gly Ser Ser Thr His Leu Val Leu Thr Leu Gly Asp Gly Val Asp Gly Phe Thr

            1241                 1256                 1271
TTA GAC ACA AAC TTG GGC GAA TTC ATC TTG ACT CAT CCT AAC TTA AGA ATT CCG
Leu Asp Thr Asn Leu Gly Glu Phe Ile Leu Thr His Pro Asn Leu Arg Ile Pro

   1286                 1301                 1316                 1331
CCT CAA AAG GCC ATC TAC TCA ATT AAT GAA GGT AAC ACC CTC TAC TGG AAC GAG
Pro Gln Lys Ala Ile Tyr Ser Ile Asn Glu Gly Asn Thr Leu Tyr Trp Asn Glu

            1346                 1361                 1376
ACT ATA AGA ACA TTT ATT GAG AAA GTC AAA CAA CCC CAA GCA GAC AAC AAC AAC
Thr Ile Arg Thr Phe Ile Glu Lys Val Lys Gln Pro Gln Ala Asp Asn Asn Asn

1391                 1406                 1421                 1436
AAG CCT TTC TCG GCT AGG TAT GTT GGA TCC ATG GTT GCT GAT GTT CAC AGG ACG
Lys Pro Phe Ser Ala Arg Tyr Val Gly Ser MET Val Ala Asp Val His Arg Thr

       1451                 1466                 1481                 1496
TTT CTT TAC GGT GGC CTT TTC GCA TAC CCT TGC GAC AAG AAG AGC CCC AAC GGA
Phe Leu Tyr Gly Gly Leu Phe Ala Tyr Pro Cys Asp Lys Lys Ser Pro Asn Gly

            1511                 1526                 1541
AAA CTG AGG TTG CTT TAT GAG GCC TTC CCA ATG GCT TTC TTA ATG GAA CAA GCA
Lys Leu Arg Leu Leu Tyr Glu Ala Phe Pro MET Ala Phe Leu MET Glu Gln Ala
```

Fig. 8B

30

```
       1556                     1571                    1586                       1601
  GGG GGA AAA GCG GTC AAC GAT CGC GGA GAG AGA ATC TTG GAT TTG GTG CCA AGT
  Gly Gly Lys Ala Val Asn Asp Arg Gly Glu Arg Ile Leu Asp Leu Val Pro Ser

                  1616                    1631                    1646
  CAT ATC CAT GAC AAA TCT TCT ATT TGG TTG GGT TCT TCA GGT GAA ATT GAC AAA
  His Ile His Asp Lys Ser Ser Ile Trp Leu Gly Ser Ser Gly Glu Ile Asp Lys

  1661                   1676                     1695      1705      1715      1725
  TTT TTA GAC CAT ATT GGC AAG TCA CAG TAGTTCAATG ATCGCCTTCT TTTCTTATTT TCTTTGTTCT
  Phe Leu Asp His Ile Gly Lys Ser Gln

         1735      1745      1755      1765      1775      1785      1795
  GTACTTTAGT ACGCGAAAAA AAAAAATCTG TATATGTCCT TATATATATA TATATTTATA TATATATATG

         1805      1815      1825      1835      1845      1855      1865
  TGTATGTATG TGTACCGTAA GCATTACTCC TTCTAATAAT GAAAATTCTT AGGAAAAGAG AAAGGAAGTA

         1875      1885      1895      1905      1915      1925      1935
  GCGAATGGAA TGGGATGGAA GTTTTAAAGA ACATTAGAAT TTATCCTTTG TCAAACTTCA TCACATCAAC

         1945      1955      1965      1975      1985      1995      2005
  CAAGAACTAT ATAAACCTAC CAAATGAATT AAGAAACCTA ATTAGTGAAG AGCAGGAGAG TAAACTAGGG

         2015      2025      2035      2045      2055      2065      2075
  TTCTTGCACA TCATTGAAAG TGATTTTAAA CCTTCGGTAG CGCTGCAAAA GTTGGTGAAT TGTACTACGG

         2085      2095      2105      2115      2125      2135      2145
  GGGACGAAAA GATCCTAATC ATAGATATAG TATCAATATG GTCCCAACAA AAGCAAAGAC AGCATGGCGC

         2155      2165      2175      2185      2195
  GATCTACATG AATTCGCTAT CTTGCATAAA CATCACGGGA TTAATCGTAT TTCTAGA
```

Fig. 8C

31

Fig. 9A

yeast fdp

Met Pro Thr Leu Val Asn Gly Pro Arg Arg Asp Ser Thr Glu Gly Phe Asp Thr Asp Ile

Ile Thr Leu Pro Arg Phe Ile Ile Glu His Gln Lys Gln Phe Lys Asn Ala Thr Gly Asp

Phe Thr Leu Val Leu Leu Asn Ala Leu Gln Phe Ala Phe Lys Phe Val Ser His Thr Ile

Arg Arg Ala Glu Leu Val Asn Leu Val Gly Leu Ala Gly Ala Ser Asn Phe Thr Gly Asp

Gln Gln Lys Lys Leu Asp Val Leu Gly Asp Glu Ile Phe Ile Asn Ala Met Arg Ala Ser

Gly Ile Ile Lys Val Leu Val Ser Glu Glu Gln Glu Asp Leu Ile Val Phe Pro Thr Asn

Thr Gln Ser Tyr Ala Val Cys Cys Asp Pro Ile Asp Gly Ser Ser Asn Leu Asp Ala Gly

Val Ser Val Gly Thr Ile Ala Ser Ile Phe Arg Leu Leu Pro Asp Ser Ser Gly Thr Ile

Asn Asp Val Leu Arg Cys Gly Lys Glu Met Val Ala Ala Cys Tyr Ala Met Tyr Gly Ser

Ser Thr His Leu Val Leu Thr Leu Gly Asp Gly Val Asp Gly Phe Thr Leu Asp Thr Asn

Leu Gly Glu Phe Ile Leu Thr His Pro Asn Leu Arg Ile Pro Pro Gln Lys Ala Ile Tyr

Ser Ile Asn Glu Gly Asn Thr Leu Tyr Trp Asn Glu Thr Ile Arg Thr Phe Ile Glu Lys

Fig. 9A (cont'd.)

```
                                    250                                    260
Val Lys Gln Pro Gln Ala Asp Asn Asn Asn Lys Pro Phe Ser Ala Arg Tyr Val Gly Ser

                                    270                                    280
Met Val Ala Asp Val His Arg Thr Phe Val Tyr Gly Gly Leu Phe Ala Tyr Pro Cys Asp

                                    290                                    300
Lys Lys Ser Pro Asn Gly Lys Leu Arg Leu Leu Tyr Glu Ala Phe Pro Met Ala Phe Leu

                                    310                                    320
Met Glu Gln Ala Gly Gly Lys Ala Val Asn Asp Arg Gly Glu Arg Ile Leu Asp Leu Val

                                    330                                    340
Pro Ser His Ile His Asp Lys Ser Ser Ile Trp Leu Gly Ser Ser Gly Glu Ile Asp Lys

Phe Leu Asp His Ile Gly Lys Ser Gln
```

EP 0 245 481 B1

Fig. 9B

pig fdp

                                          10                                        20
Thr Asp Gln Ala Ala Phe Asp Thr Asn Ile Val Thr Leu Thr Arg Phe Val Met Glu Gln

                                          30                                        40
Gly Arg Lys Ala Arg Gly Thr Gly Glu Met Thr Gln Leu Leu Asn Ser Leu Cys Thr Ala

                                          50                                        60
Val Lys Ala Ile Ser Thr Ala Val Arg Lys Ala Gly Ile Ala His Leu Tyr Gly Ile Ala

                                          70                                        80
Gly Ser Thr Asn Val Thr Gly Asp Gln Val Lys Lys Leu Asp Val Leu Ser Asn Asp Leu

                                          90                                        100
Val Ile Asn Val Leu Lys Ser Ser Phe Ala Thr Cys Val Leu Val Thr Glu Glu Asp Lys

                                          110                                       120
Asn Ala Ile Ile Val Glu Pro Glu Lys Arg Gly Lys Tyr Val Val Cys Phe Asp Pro Leu

                                          130                                       140
Asp Gly Ser Ser Asn Ile Asp Cys Leu Val Ser Ile Gly Thr Ile Phe Gly Ile Tyr Arg

                                          150                                       160
Lys Asn Ser Thr Asp Glu Pro Ser Glu Lys Asp Ala Leu Gln Pro Glu Arg Asn Leu Val

                                          170                                       180
Ala Ala Gly Tyr Ala Leu Tyr Gly Ser Ala Thr Met Leu Val Leu Ala Met Val Asn Gly

                                          190                                       200
Val Asn Cys Phe Met Leu Asp Pro Ala Ile Gly Glu Phe Ile Leu Val Asp Arg Asn Val

                                          210                                       220
Lys Ile Lys Lys Lys Gly Ser Ile Tyr Ser Ile Asn Glu Gly Tyr Ala Lys Glu Phe Asp

EP 0 245 481 B1

Fig. 9B (cont'd.)

                                                230                                              240
Pro Ala Ile Thr Glu Tyr Ile Glu Arg Lys Lys Phe Pro Pro Asp Asn Ser Ala Pro Tyr

                                                250                                              260
Gly Ala Arg Tyr Val Gly Ser Met Val Ala Asp Val His Arg Thr Leu Val Tyr Gly Gly

                                                270                                              280
Ile Phe Met Tyr Pro Ala Asn Lys Lys Ser Pro Lys Gly Lys Leu Arg Leu Leu Tyr Glu

                                                290                                              300
Cys Asn Pro Met Ala Tyr Val Met Glu Lys Ala Gly Gly Leu Ala Thr Thr Gly Lys Glu

                                                310                                              320
Ala Val Leu Asp Ile Val Pro Thr Asp Ile His Gln Arg Ala Pro Ile Ile Leu Gly Ser

                                                330
Pro Glu Asp Val Thr Glu Leu Leu Glu Ile Tyr Gln Lys His Ala

EP 0 245 481 B1

Ndel
|
5'         Met Pr o...... 3'
TCATACTACCACACA|TATGCC AACT.........(FDP ase)

Cut Ndel
Mung Bean Exonuclease
Cut Xhol

TGCCAACT..........

TCGAGACACAAGCA      Xhol/Sphl ADAPTER
   CTGTGTTCGT

ligate ADAPTER
CLOSE

Xhol               Sphl
C|TCGAGACACAAGCATG|CCAACT
GAGCT|CTGTGTTC|GTACGGTTGA

FIG. 10

FIG. II

EcoRV

GAT ACC GAT ATC ATC ACT CTT  DNA

Asp Thr Asp Ile Ile Thr Leu  AMINO ACIDS

17  18  19  20  21  22  23  RESIDUE

FIG. 12

FIG. 13

FIG. 14 a

FIG. 14 b

EP 0 245 481 B1

FIG. 14c

EP 0 245 481 B1

FIG. 15A

FIG. 15B

EP 0 245 481 B1

FIG. 17

FIG. 16

FIG. 18

* THESE SITES ARE NOT UNIQUE

Fig. 19

GGATCCGAAAGTTGTATTCAACAAGAATGCGCAAATATGTCAACGTATTTGGAAGTCAAC

TTATGTGCGCTGCTTTAATGTTTTCTCATGTAAGCGGACGTCGTCTATAAACTTCAA

ACGAAGCTAAAAGGTTCATAGCGCTTTTTTCTTTGTCTGCACAAAGAAATATATATTAAA

TTAGCACGTTTTCGCATAGAACGCAACGAACGCAACTGCACAATGCCAAAAAAAGTAAAA

                                        CLA1
                                         ↓
GTGATTAAAAGAGTTAATTGAATAGGCAATCTCTAAATGTATCGATACAACCTTGGCACT

CACACGTGGGACTAGCACAGACTAAATTTATGATTCTGGTCCCTGTTTTCGAAGAG

ATCGCACATGCCAAATTATCAAATTGGTCACCTTACTTGGCAAGGCATATACCCATTTGG

GAATAAAGGGTAAACACTTTGAATTGTCGAAATGAAACGTATATAAGCGCTGATGTTTTG

CTAAGTCGAGGTTAGTATGGCTTCATCTCTCATGAGAATAAGAACAACAAGAAATAGAGC

                      MetPheLysSerValValTyr........
                       |  |  |  |  |  |  |
AAGCAAATTCGAGATTACCA<u>ATG</u>TTTAAATCTGTTGTTTATTCAATTTTAGCCGCTTCT

                Kpnl
                 ↓
TTGGCCAATGCAGGTACC

45

FIG. 20

FIG. 21a

FIG. 21b

FIG. 22a

FIG. 22 b

FIG. 23